# EUROPEAN PATENT APPLICATION

(11) **EP 4 481 386 A1**
(43) Date of publication of application: **25.12.2024**
(21) Application number: 23180126.7
(22) Date of filing: 19.06.2023
(51) Int. Cl.: G01N 33/543, G01N 33/74, G01N 33/553, G01N 27/327, A61B 5/00, A61B 10/00

(54) **IMPLANTABLE APPARATUSES, BIOSENSOR DEVICES, SYSTEMS AND METHODS**

(71) Applicant: Impli AG, Luzern (CH)
(72) Inventor: Rosa, Bruno Miguel Gil, London (GB); Hartle, Heathcliff J., Lausanne (CH); Fard, Ghazal Hatami, London (GB); Anastasova-Ivanova, Salitsa, London (GB); Schaffgotsch, Anna-Luisa Graefin, London (GB)
(74) Representative: Wallinger Ricker Schlotter Tostmann

(57) **Abstract**

The present invention is directed to biosensor devices, implantable apparatuses, and systems, the implantable apparatus including glass and the biosensor device using aptamers and being, in particular battery-less, and to methods for fabrication or for operating the apparatus/device, apparatuses and systems.

## Description

The present invention is directed to biosensor devices, implantable apparatuses, and systems, which comprise the devices and/or implantable apparatuses, and to methods for fabrication or for operating the apparatus/device, apparatuses and systems.

A biosensor is a device that combines a biological component with a physicochemical detector, or respectively a biochemical sensor, to detect and analyze specific biological or biochemical analytes (hereinafter: biomarkers or biological quantities). It is designed to convert a biological response, such as the interaction between a biomolecule and a target analyte, into a measurable signal.

The biological component of a biosensor can be various biological elements, including enzymes, antibodies, nucleic acids, or whole cells. These components are selected based on their ability to recognize and interact with the target analyte of interest. When the target analyte binds to the biological component, it triggers a specific biochemical or biophysical change.

The physicochemical detector (here also: biosensor device), often referred to as the transducer, converts the biological response into a measurable signal. This can be achieved through various mechanisms, such as electrical, optical, or electrochemical methods. The transducer produces a signal proportional to the concentration or presence of the target analyte, allowing for quantitative or qualitative analysis.

Biosensors have a wide range of applications in various fields, including healthcare, environmental monitoring, food safety, and biotechnology. They can be used to detect and monitor biomarkers for disease diagnosis, measure glucose levels in diabetes management, detect environmental pollutants, or analyze DNA sequences. Biosensors offer advantages such as rapid detection, sensitivity, selectivity, portability, and real-time monitoring, making them valuable tools in many industries.

The invention is, in particular, related to an implantable apparatus for sensing at least one biological quantity inside the body of an animal or a human.

An implantable apparatus, in this context, is an apparatus, which is configured for being implanted, in particular subcutaneously implanted, in particular completely implanted, into the body of an animal or a human.

An implantable apparatus for sensing a biological quantity inside the body of an animal or a human is also referred to as an implantable biosensor. Implantable biosensors are designed to monitor and measure specific biological quantities, i.e. biological parameters, within the body.

Implantable biosensors can be used for a wide range of applications, including healthcare, biomedical research, veterinary medicine. They may collect data and/or provide real-time data on various biological quantities, i.e. physiological or biochemical parameters, enabling patients, healthcare professionals and researchers to monitor conditions, diagnose diseases, and optimize treatment strategies.

Examples of implantable biosensors used for sensing biological quantities include: Implantable glucose sensors, which are used for continuous monitoring of glucose levels in individuals with diabetes. They provide accurate and frequent glucose readings, eliminating the need for regular finger-prick tests. Implantable pressure sensors, which are used to measure pressure within different parts of the body. For instance, implantable intracranial pressure sensors can monitor pressure in the brain, helping to diagnose and manage conditions such as hydrocephalus or traumatic brain injuries. Implantable temperature sensors, which are used to measure body temperature from within the body. They can be used for various purposes, including monitoring core body temperature during surgeries or tracking temperature changes in specific tissues. Implantable pH sensors, which measure the acidity or alkalinity of a solution and which can be used to monitor pH levels in different body fluids ortissues, providing valuable information for diagnosing and managing conditions such as acidosis or alkalosis. Implantable oxygen sensors, which measure the oxygen levels within body tissues or fluids. They can be used to monitor tissue oxygenation in critical care settings, assess wound healing, or optimize oxygen delivery during surgery.

There are also wearable and implantable biosensors that are not directly related to healthcare. These types of biosensors are designed for various purposes outside the traditional healthcare field. Implantable biosensors can be used to monitor physiological parameters in athletes during training and competition. These sensors can track metrics such as heart rate, body temperature, hydration levels, and lactate levels. The data collected can help athletes optimize their training, prevent overexertion, and enhance performance. Another application is related to biohacking and quantified self: some individuals engage in biohacking or the quantified self movement, which involves tracking and optimizing various aspects of their own biology. Implantable biosensors can be used to monitor parameters like body temperature, sleep patterns, hormone levels, or even brain activity. This data can provide insights into personal health, habits, and performance optimization. Another application is related to industrial monitoring. In industrial settings, implantable biosensors can be used for worker safety and environmental monitoring. For example, sensors implanted in workers can monitor exposure to toxic substances, radiation levels, or other occupational hazards. This helps ensure a safe working environment and enables early detection of potential health risks. Another application is related to animal tracking and research. Implantable biosensors are also used in wildlife and animal research for tracking, monitoring behavior, and collecting physiological data. These sensors can be implanted in animals to track migration patterns, habitat use, breeding behavior, or to monitor specific biological parameters. The data collected helps in conservation efforts, understanding animal behavior, and ecological research. While these examples involve implantable biosensors for non-healthcare purposes, they still involve monitoring and collecting biological/physiological quantities, including physical parameter being immediately relevant for biology, like a radiation exposure etc. The applications may differ, but the underlying principles of using biosensors to gather biological data, in particular real-time data, remain consistent.

Implantable biosensors offer the advantage of a simplified monitoring, in particular a continuous, real-time monitoring of biological quantities, allowing for early detection of abnormalities and timely intervention. Due to their small size and their biocompatibility they are designed to minimize tissue damage or discomfort. Additionally, they may utilize wireless communication technology to transmit data to external devices for analysis and interpretation.

Access or use of assisted reproductive technology (ART) is highly underrepresented in the global population. The European Society of Human Reproduction and Embryology estimates the optimal utilization of *in vitro* fertilization (IVF) / intracytoplasmic sperm injection (ICSI) to be 1,500 couples per million population per year (Andersen AN, et.al.; European IVF-monitoring programme (EIM), European Society of Human Reproduction and Embryology (ESHRE). Assisted reproductive technology in Europe, 2001. Results generated from European registers by ESHRE. Hum Reprod. 2005 May;20(5):1158-76. doi: 10.1093/humrep/deh755. Epub 2005Jan 21. PMID: 15665021.) with multiple rounds of ART cycles for a successful outcome. However, a study in 2011 found that the overall global utilization rate of was only 477 cycles per million population with an estimated 2.0 million ART cycles resulting in 0.5 million babies (Adamson GD, et.al., V. International Committee for Monitoring Assisted Reproductive Technology: World Report on Assisted Reproductive Technology, 2018.).

The low success rate of IVF is perceived as the result of transfer of IVF-derived embryos into the non-receptive uterus. One cause of this low rate could be the high levels of estrogen resulting from gonadotropin administration to retrieve multiple eggs, rendering the uterus refractory. Thus, uterine receptivity coordinated by the interactions of progesterone with estrogen is critical to successful implantation and pregnancy outcome. The results from an experiment by Ma et al., using a delayed implantation model in mice provides evidence that the levels of estrogen within a very narrow range are critical determinants for transforming uterine receptivity to a refractory state, suggesting that the uterus is extremely sensitive to estrogen levels with respect to implantation (Ma WG, Song H, Das SK, Paria BC, Dey SK. Estrogen is a critical determinant that specifies the duration of the window of uterine receptivity for implantation. Proc Natl Acad Sci U S A. 2003 Mar 4;100(5):2963-8. doi: 10.1073/pnas.0530162100. Epub 2003 Feb 24. PMID: 12601161; PMCID: PMC151449).

Further, ART has multiple risks to the female such as hyperstimulation of the ovaries and gestational pregnancies. In Denmark, 1.2% of women encountered ovarian hyperstimulation syndrome (OHSS) of which 75% were hospitalized for more than 24 hours. This has led the industry to build more precision into their treatments. A study of 2,700 women showed that individualized stimulation of the ovaries showed better outcome. However reliable and accurate biomarkers are not frequently captured (Roudebush, W.E., Kivens, W.J. and Mattke, J.M. (2008) "Biomarkers of ovarian reserve," Biomarker Insights, 2008 Apr 16;3:259-268. doi: 10.4137/bmi.s537. PMID: 19578510; PMCID: PMC2688347.). The processes are not standardized between clinics and variability remains whether a clinic promoted monitoring using blood markers or if the clinic solely relies on ultrasound scans.

Currently, IVF treatments require females to undergo blood analysis every two days. This causes major inconvenience; however, it is necessary to get more accurate results and outcomes of the procedure and adapt clinical protocols during treatment to increase the safety of treatment. Females are also using other technologies to track hormone levels such as urine strips to measure the presence of luteinizing hormone (LH), capturing saliva to measure progesterone levels and temperature with wearables to evidence ovulation cycles. Depending on the progression of the women the female is monitored up to 2-3 times per week and sometimes more frequently. However, there are few alternatives to accurately monitoring hormone levels and those are not performed in real-time. The lack of more precise data also limited the optimization of precision medicine in ART cycles and fertility. Hormone levels in women are not understood in prolonged real-time and the impact this could have on the female community could be revolutionary not only in ART but many other fertility-related problems such as assisted conception or the detection of specific ovarian conditions. By measuring the levels of estrogen, progesterone and luteinising hormone in real-time; the fertility window could be easily and accurately identified. Measuring estradiol and progesterone are the most reliable markers of the ovarian events as they are both direct markers that emanate from the ovary (Blackwell, L., Cooke, D. and Brown, S. (2018) "Self-Monitoring of Fertility Hormones: A New Era for Natural Family Planning?" Linacre Q. 2018 Feb;85(1):26-34. doi: 10.1177/0024363918756387. Epub 2018 Mar 28. PMID: 29970935; PMCID: PMC6027114).

. In terms of IVF, baseline hormone levels can be used to determine the women's natural fertility profile and decide on a specific ovarian stimulation method. The technology can also monitor the individual's hormone response and determine the appropriate medication dose and date of administration. An example of this is human chorionic gonadotropin (hCG) is administered on the sixth day of a sustained increase in serum estradiol levels (Liu Y, Li J, Zhang W, Guo Y. Association between serum estradiol level on the hCG administration day and neonatal birthweight after IVF-ET among 3659 singleton live births. Sci Rep. 2021 Mar 16;11(1):6084. doi: 10.1038/s41598-021-85692-7. PMID: 33727635; PMCID: PMC7966761).

Further, assessment of LH pulsatility is important for the clinical diagnosis of reproductive disorders, but current methods are hampered by frequent blood sampling coupled to expensive serial immunochemical analysis. Normal reproductive function is governed by a highly orchestrated pattern of hormonal feedback across the hypothalamic-pituitary-gonadal (HPG) axis (Barbieri, R. L. The endocrinology of the menstrual cycle. Methods Mol. Biol. 1154, 145-169 (2014) doi: 10.1007/978-1-4939-0659-8_7. PMID: 24782009). The pulsatile release of LH, estradiol and progesterone is a critical element for downstream regulation of sex steroid hormone synthesis and the production of mature oocytes. Altered patterns of hormone pulse secretion have been linked to hypothalamic dysfunction, resulting in numerous reproductive disorders, including polycystic ovary syndrome (PCOS) (Bachelot, A. et al. Luteinizing hormone pulsatility in patients with major ovarian hyperandrogenism. J. Endocrinol. Invest. 30, 636-646 (2007) doi: 10.1007/BF03347443. PMID: 17923794) hypothalamic amenorrhoea (Touraine, P. et al. Resumption of luteinizing hormone pulsatility and hypogonadotropic hypogonadism after endoscopic ventriculocisternostomy in a hydrocephalic patient. Fertil. Steril. 76, 390-393 (2001)) and delayed or precocious puberty. It is not currently feasible in routine clinical practice to measure hormone pulsatility to determine altered secretion patterns because to do so is extremely resource intensive. Peripheral blood sampling is necessary every 10 min for at least 8 h, and serial analysis by immunochemical assay is expensive. However human studies from specialist clinical research groups (Prague, J. K. et al. Neurokinin 3 receptor antagonism as a novel treatment for menopausal hot flushes: a phase 2, randomised, double-blind, placebocontrolled trial. Lancet 389, 1809-1820 (2017), doi: 10.1016/S0140-6736(17)30823-1. Epub 2017 Apr 3. PMID: 28385352; PMCID: PMC5439024. Dhillo, W. S. et al. Kisspeptin-54 stimulates the hypothalamic-pituitary gonadal axis in human males. J. Clin. Endocrinol. Metab. 90, 6609-6615 (2005) Epub 2005 Sep 20. PMID: 16174713.) have shown that there is a potential therapeutic benefit to women undergoing IVF and menopause to have the ability to more accurately monitor hormone concentration fluctuations. There are three major issues currently preventing widespread clinical hormone concentration profile measurement and its pulsatility analysis: (1) Hormone concentration profile's resolution is restricted by the sampling protocol and immunochemical assay-there is no method capable of real-time monitoring of LH pulsatility. (2) Measurement of hormonal concentration profile is restricted by cost when serial clinical chemiluminescent immunoassays are used (∼£20 per sample, 50 samples required for one patient). (3) Hormonal pulsatility analysis is challenging as it typically requires advanced algorithms that can appropriately and efficiently account for the inherent biological variation, pulse-by-pulse variability and physiological factors impacting on hormone secretion and calculation, including elimination. There is a clear unmet medical need for better translational technologies that could enable routine clinical LH pulsatility analysis for patients with reproductive disorders.

A biosensor device is, in particular, a device that uses biological components, such as enzymes, antibodies, or living cells, to detect the presence or concentration of a specific substance, such as a chemical or biological molecule. Biosensors typically consist of three components: a biological recognition element, a transducer, and a signal processing system.

The biological recognition element is the part of the biosensor that interacts with the target molecule and generates a signal. For example, an enzyme biosensor might use an enzyme to catalyze a reaction that produces a detectable product, while an antibody biosensor might use an antibody to bind to a specific antigen and generate a signal. The transducer is the part of the biosensor that converts the signal generated by the biological recognition element into a measurable output signal, such as an electrical, optical, or acoustic signal. The signal processing system is the part of the biosensor that interprets the output signal and provides information about the concentration or presence of the target molecule. According to a preferred aspect of the invention, the biological recognition element is an aptamer, in particular a biosensor membrane containing a layer of aptamers.

Intrabody monitoring offers a direct connection to the body processes executed at the cellular and tissue levels. Implantable biosensors will be able to accurately detect local levels of different biomarkers.

Implantables have been significantly established in contraception, where intrauterine devices have been on the market since the 19^{th} century and have been widely available commercially since the 1970s. Further, the contraceptive implant which is subdermal launched in the US in 2006. Although previous implantables were used in this market, biosensor devices, in particular sub-dermal biosensor devices have never been used in infertility care.

Thus, there is a need for improved technologies that could enable detection, particularly real time detection, of hormones.

In an aspect, the present invention relates to a biosensor device for sensing at least one hormone, comprising:
(a) at least one metal electrode surface; and
(b) a biosensor membrane including at least one aptamer which is attached to the metal electrode surface, wherein the aptamer:
   (i) is capable of binding the at least one hormone; and
   (ii) is modified with one or more functional group for attaching the at least one aptamer to the metal electrode surface.

Preferably, the biosensor device is configured for sensing the at least one hormone in a body fluid, in particular an interstitial fluid.

Preferably, the biosensor device is an implantable device configured to be fully implantable into the body of an animal or human, in particular subcutaneously. Preferably, the biosensor device is a wearable device, in particular configured to be used in contact with the skin of an animal or a human.

Preferably, the at least one hormone is selected from the group consisting of estradiol, luteinizing hormone (LH), progesterone, and any combination thereof.

Preferably, the biosensor device further comprises a carrier, particularly a biocompatible glass carrier, which preferably is transparent, or preferably is opaque.

Preferably, the aptamer is a single stranded nucleic acid molecule, and/or preferably a DNA or RNA molecule, binding specifically with high affinity and specificity.

Preferably, the aptamer has a length of about 25 to 70 nucleotides, preferably about 30 to about 65 nucleotides.

Preferably, the functional group for attaching the at least one aptamer to the metal electrode surface is a thiol group.

Preferably, the functional group for attaching the at least one aptamer to the metal electrode surface is present at the end of the aptamer.

Preferably, the aptamer is a single stranded nucleic acid molecule, preferably a DNA molecule, and the functional group for attaching the at least one aptamer to the metal electrode surface is present at the 3' end or 5' end, preferably the 5' end.

Preferably, the aptamer is capable of binding at least one hormone selected from the group consisting of estradiol, luteinizing hormone (LH), and progesterone.

Preferably, the aptamer is one or more selected from group consisting of SEQ ID NO: 1, SEQ ID NO: 2, SEQ ID NO: 3, and SEQ ID NO: 4.

Moreover, the invention is related to the biosensor device according to the invention for use in diagnosis, particularly in sensing at least one hormone, more particularly for monitoring hormones during assisted reproductive technology (ART), during menopause, in a hormone disorder such as polycystic ovary syndrome (PCOS), in endometriosis and/or during hormone treatment.

Moreover, the invention relates to the use of the biosensor device according to the invention for *in vitro* diagnosis, particularly for sensing of at least one hormone.

Moreover, the invention relates to an implantable apparatus for being implanted, in particular subcutaneously implanted, into the body of an animal or human, comprising
- a biosensor device according to the invention, and
- an electronic control device for controlling the biosensor device, in particular for generating, collecting, and preferably, encrypting, measurement data obtained by the sensing of the biosensor device.

Moreover, the invention relates to a system for sensing at least one hormone, comprising
- the implantable apparatus according to the invention, which additionally comprises a communication device, in particular NFC (Near Field Communication) device for wireless data communication with an external computer device being separate from the implantable apparatus, and
- the external computer device comprising a communication device, in particular NFC device, for wireless data communication with the implantable apparatus.

Moreover, the invention relates to a method for operating a biosensor device according to any of the preceding claims, comprising the steps:
(a) contacting the metal electrode surface with a body fluid, particularly interstitial fluid;
(b) allowing the at least one aptamer of the metal electrode surface to bind the at least one hormone; and
(c) detecting the binding of the at least one hormone by electrically controlling the at least one metal electrode surface, preferably via differential pulse voltammetry (DPV).

Unless otherwise defined, all technical and scientific terms used herein have the same meaning as commonly understood by one of ordinary skill in the art to which this disclosure belongs.

It is noteworthy that the use of the undefined article "a" or "an" means "one or more". Thus, for example, the term "a hormone" includes the incorporation of "one" and "more than one" hormone(s).

The term "comprising" or "comprises" as used herein means "including, but not limited to". The term is intended to be open-ended, to specify the presence of any stated features, elements, integers, steps, or components, but not to preclude the presence of addition of one or more other features, elements, integers, steps, components, or groups thereof. The term "comprising" or "comprises" thus includes the more restrictive terms "consisting of" and "consisting essentially of". In one embodiment, the term "comprising" or comprises" as used throughout the application and in particular within the claims may be replaced by the term "consisting of" or "consisting essentially of".

The present invention, according to a preferred aspect, relates to a biosensor device for sensing at least one hormone, comprising at least one metal electrode surface and a biosensor membrane, the biosensor membrane comprising at least one aptamer which is attached to the metal electrode surface.

The biosensor device according to the invention improves the IVF technology, and the way treatments are administered today because it enables real time monitoring. It further leads to innovation in other female health areas where frequent and accurate hormone level capture is important. For example, polycystic ovary syndrome, endometriosis, and menopause. Applications in these areas will help clinicians with early diagnosis and deliver more accurate treatments. Moreover, the data collected with the inventive biosensor allows better predictive and personalized machine learning algorithms to be built with better capabilities and more accurate outcomes vital in clinical practice.

The biosensing membrane (also referred to as "biosensor membrane") of the biosensor device is also referred to as an "aptasensor". The aptasensor is able to detect the analyte of interest, in particular the biological quantity, accurately and repeatedly. The aptasensor is preferably prepared in a few steps electrochemically as outlined in the Examples herein. These steps preferably include: the activation of the metal electrode surface acting as the working electrode; preferably: an addition of nanoparticles-layer(s) for increased sensitivity; the addition of the functional sensing layer formed by the aptamers, and preferably: finally an addition of a protective porous biocompatible membrane. Whilst the biosensor is in contact with even a trace amount of the analyte, aptamers will bind to it and a measurement signal will be sensed and recorded. The precise quantity, in particular the amount of analyte or concentration of the analyte or the alteration, in particular time-dependent alteration, can be measured using this approach. The aptasensor is preferably calibrated and/or otherwise validated prior to its deployment.

In principle, biosensing of analytes via electrochemical sensors can be obtained using, antibodies, aptamers, and protein receptors. The advantage of aptamers is that they can bind to the analytes of interest in a repeatable manner, while antibodies bind to the target irreversibility. Relative to other biosensors using enzymes or antibodies, aptamer's chemical simplicity, stability, specificity and selectivity enable continuous *in vivo* monitoring. The advantage including an equal or superior affinity and specificity to the target, a smaller size, easier modification and immobilization, better stability, and higher reproducibility allowed the development of highly specific biosensors of the invention. The inventors found that using aptamers provides a significant and unexpected advantage with respect to longevity of the biosensor device over other biological recognition elements for detecting biomarkers, in particular hormones, in vivo.

The integration of aptamers onto microelectrodes, which are small enough to facilitate implantation, results in challenges such as the limited current signal due to low aptamer density on the electrode surface. This in turn results in a low signal to noise ratio (SNR) which may be exacerbated, in embodiments of the invention, by limitations of a low power communication device, in particular NFC device. To boost SNR, the inventors developed a strategy involving the use of nanoparticles, in particular gold and/or platinum nanoparticles, carbon nanotubes or carbon nanoparticles. As a result of using such nanoparticles, the overall sensing surface is significantly increased and the SNR is improved. The incorporation of nanoparticles into the sensing layer significantly enhances the sensitivity of the electrochemical sensor. These nanoparticles are utilized for various purposes, such as modifying the electrode surface, labeling specific target molecules for detection, amplifying signals, and acting as catalysts for ongoing chemical reactions. Additionally, nanoparticles offer excellent biocompatibility and provide a larger surface area over the electrodes, thereby improving electron transfer capability, facilitating the immobilization of bioactive substances on the electrode surface, and reducing the time required for detection. The nanoparticles employed can vary in size (within the nanometer range), shape (spherical, cylindrical, planar, etc.), and composition, including noble metal nanomaterials like gold and silver, semiconductor materials such as quantum dots, carbon-based nanomaterials like carbon nanotubes and graphene oxide, as well as composite nanomaterials. Depending on the specific nanomaterial and substrate surface, there are multiple methods available for depositing nanoparticles, such as dip coating, spin coating, solvent evaporation, chemical vapor deposition, and transfer printing.

Preferably, the biosensor device is configured for sensing the at least one hormone in a physiological fluid or a body fluid such as blood, blood serum, salvia, urine, sweat, tears, mucus and vaginal discharge, uterine wall and menstrual blood, or interstitial fluid, peferably: interstitial fluid.

As stated above, intrabody monitoring offers a direct connection to the body processes executed at the cellular and tissue levels. Implantable biosensors will be able to accurately detect local levels of different biomarkers. Thus, in one embodiment, the biosensor device is an implantable apparatus configured to be fully implantable into the body of an animal or human, in particular subcutaneously.

An "aptamer" as used herein refers to a short single-stranded ribonucleic acid (RNA), deoxyribonucleic acid (DNA) or xeno nucleic acid (XNA) which binds a specific target molecule with affinity. The high affinity is basically achieved by a complex and diverse secondary and tertiary structure through intramolecular partial duplex formation of the single-stranded nucleic acid resulting in a defined 3-dimensional structure. Typically, aptamers have a size of about 20 to 100 nucleotides. Targets molecules can include small molecules, heavy metal ions, larger ligands such as proteins and even whole cells. Aptamers might also be modified by specific functional groups to enhance their function and/or compatibility with larger engineered molecular systems. Aptamers can be applied in a wide variety of fields including but not limited to sensing, therapeutic, reagent production, and engineering categories. For example, the aptamer is used as a sensing molecule to bind specific biomolecules, such as hormones, which are present in a body fluid, particularly in interstitial fluid.

In the context of the invention, any aptamer can be used which is capable of binding the hormone to be sensed. The skilled person knows how to develop aptamers which specifically bind a given hormone including but not limited to systematic evolution of ligands by exponential enrichment (SELEX). SELEX is a multi-step process aimed at identifying and optimizing short, single-stranded DNA or RNA molecules, i.e., aptamers, that can bind to specific target molecules with high affinity and specificity. The SELEX process is known to the skilled person and may vary slightly depending on the specific goals, target molecule, and resources available to the researchers.

For example, the SELEX process might involve the following steps:
1) Target identification: The first step is to identify the target molecule to which the aptamer will bind. This can be a small molecule, protein, or even a whole cell.
2) Library generation: A large library of random DNA or RNA sequences, typically containing billions of different sequences, is synthesized. These sequences serve as the starting point for aptamer selection.
3) Selection: The real SELEX process begins. The library of random sequences is incubated with the target molecule, allowing the aptamers with binding affinity for the target to bind.
4) Partitioning: The bound aptamers are separated from the unbound ones. Various techniques such as filtration, affinity chromatography, or magnetic separation can be employed for this purpose and are known to the person skilled in the art.
5) Amplification: The bound aptamers are eluted and amplified through a process called polymerase chain reaction (PCR) or reverse transcription PCR (RT-PCR). This step increases the concentration of the selected aptamers for the subsequent rounds of selection.
6) Iterative rounds: Steps 3-5 are repeated for several rounds, typically 8-15 rounds, to enrich the pool of aptamers with high affinity and specificity for the target. The stringency of the selection conditions is gradually increased in each round to favor the selection of the best aptamers.
7) Sequencing and analysis: After the desired number of selection rounds, the enriched aptamer pool is sequenced to determine the DNA or RNA sequences of the selected aptamers. Bioinformatics analysis is then performed to identify common motifs or structural features among the aptamers.
8) Aptamer optimization: Selected aptamer sequences are synthesized and further optimized to improve their binding properties, stability, and specificity. This can be achieved through chemical modifications, truncation, or mutagenesis.
9) Validation and characterization: The optimized aptamers are tested to confirm their binding affinity, specificity, and functionality using various techniques such as surface plasmon resonance, fluorescence assays, or cell-based assays. These methods are known to the skilled person.
10) Applications: Finally, the validated aptamers can be used in a wide range of applications, including diagnostics, therapeutics, biosensing, drug delivery, and biomarker discovery.

Preferably, the aptamer is a single stranded nucleic acid molecule, preferably a DNA molecule. For example, the aptamer is an oligonucleotide.

Preferably, the aptamer has a length of about 25 to 70 nucleotides, preferably about 30 to about 65 nucleotides.

As stated herein, the aptamers can be modified with functional groups to increase their functionality. Methods for modifying the aptamers with functional groups may vary and generally depend on the specific functional group and/or the attachment site of the functional group on the aptamer. These methods involve basic chemistry and are generally known in the art. In one embodiment, the aptamer is modified with a functional group for attaching the at least one aptamer to the metal electrode surface. In one embodiment, the aptamer is modified with one or more functional groups for attaching the at least one aptamer to the metal electrode surface. In principle, any functional group which enables attachment of the aptamer to the metal electrode surface can be used and the functional group may depend on the nature and composition of the surface of the metal electrode. In a specific embodiment, the functional group is a thiol-group (-SH). The thiol-group on the aptamer allows for the attachment of the aptamer to the metal electrode surface, in particular a gold electrode surface. This is called a self-assembled monolayer (SAM). In principle, the functional group can be present at any site within the aptamer as long as the binding activity of the aptamer to the target analyte is not impaired or compromised. The exact location of the functional group might depend on the specific aptamer and the surface of the metal electrode.

A "self-assembled monolayer" (SAM) refers to a single layer of molecules that spontaneously organize themselves into a well-ordered pattern on a solid substrate. Without being bound to a particular theory, this organization occurs through intermolecular forces such as van der Waals interactions, hydrogen bonding, or electrostatic interactions. SAMs are commonly formed by immersing a substrate into a solution containing the desired molecules, allowing them to adsorb and self-assemble on the surface. SAMs play a crucial role in crosslinking processes by providing a template for the attachment of crosslinking agents or polymers. The functional groups present on the SAM surface can react with the crosslinking agents, enabling the formation of covalent bonds between the molecules. This crosslinking process enhances the stability and mechanical properties of the resulting material. SAMs provide a well-defined and controllable surface architecture that can be utilized for crosslinking reactions, enabling the development of advanced materials with tailored properties and improved performance.

Preferably, the functional group for attaching the at least one aptamer to the metal electrode surface is present at the end of the aptamer. For example, the aptamer is a single stranded nucleic acid molecule, preferably a DNA molecule. Then the functional group for attaching the at least one aptamer to the metal electrode surface might be present at the 3' end and/or 5' end. Preferably, the aptamer is a single stranded nucleic acid molecule, preferably a DNA molecule and the functional group for attaching the at least one aptamer to the metal electrode surface is present at the 3' end. Preferably, the aptamer is a single stranded nucleic acid molecule, preferably a DNA molecule and the functional group for attaching the at least one aptamer to the metal electrode surface is present at the 5' end. Attachment at the 5' end can, e.g., be accomplished by replacing a hydroxy group of the phosphor group of the 5' terminal nucleotide with a thiol group. Attachment at the 3' end can, e.g., be accomplished by replacing a hydroxy group of the ribose unit of the 3' terminal nucleotide with a thiol group.

Preferably, the biosensor of the present invention is configured for the sensing of at least one hormone. Hereby, the aptamers are capable of binding to the at least one hormone. Hormones to be detected include but are not limited to estradiol, luteinizing hormone, and progesterone. Preferably, the hormone to be detected is selected from the group consisting of estradiol, luteinizing hormone (LH), progesterone, and any combination thereof. Moreover, the hormone to be detected may be selected from the group consisting of estradiol, luteinizing hormone (LH), progesterone, Cortisol, FSH, Testosterone, Serotonin, Triiodothyronine T3, Tetraiodothyronine T4, Calcitonin, Insulin, Melatonin, TSH, hGH, AmH, Glucagon, and any combination thereof.

Thus, preferably, the aptamer is capable of binding at least one hormone selected from the group consisting of estradiol, luteinizing hormone, and progesterone. For example, the biosensor is for sensing estradiol. Thus, the biosensor membrane comprises at least one aptamer which is capable of binding estradiol. For example, the biosensor is for sensing luteinizing hormone. Thus, the biosensor membrane comprises at least one aptamer which is capable of binding luteinizing hormone. For example, the biosensor is for sensing progesterone. Thus, the biosensor membrane comprises at least one aptamer which is capable of binding progesterone. In another example, the biosensor is for sensing, e.g., the combination of estradiol and progesterone. Thus, the biosensor membrane comprises at least one aptamer which is capable of binding estradiol and at least one aptamer which is capable of binding progesterone. In another example, the biosensor is for sensing the combination of estradiol, luteinizing hormone, and progesterone. Thus, the biosensor membrane comprises at least one aptamer which is capable of binding estradiol, at least one aptamer which is capable of binding luteinizing hormone, and at least one aptamer which is capable of binding progesterone. Preferably, the aptamers of the biosensor membrane consist of the at least one aptamer to be sensed or combination of aptamers to be sensed. Preferably, the biosensor membrane comprises a plurality of aptamers binding a specific hormone to be sensed. In one embodiment, the plurality of aptamers to be sensed is arranged as a layer on the surface of the metal electrode.

The amount of the individual aptamer(s) capable of binding a specific hormone or the relative amounts of aptamers capable of binding to different hormones present on the biosensor membrane is variable and might depend on the desired stability and mechanical properties of the resulting material as well as the desired architecture which enables the development of advanced materials with tailored properties and improved performance.

Preferably, the aptamer is one or more selected from group of the following aptamers:
LH aptamer :
5'-TATGGTATGCTGTGTGGTATGGGGTGGCGTGCTCT-3' (SEQ ID NO: 1)

Preferably, the functional group for attaching the at least one aptamer to the metal electrode surface is a thiol group and might be present at the 3' end and/or 5' of SEQ ID NO: 1. In a preferred embodiment, the functional group for attaching the at least one aptamer to the metal electrode surface is a thiol group and is present at the 5'end.
Beta estradiol aptamers:
5'-TTTTTTTTTTTTTTTGCTTCCAGCTTATTGAATTACACGCAGAGGGTA-3' (SEQ ID NO: 2)

Preferably, the functional group for attaching the at least one aptamer to the metal electrode surface is a thiol group and might be present at the 3' end and/or 5' of SEQ ID NO: 2. Preferably, the functional group for attaching the at least one aptamer to the metal electrode surface is a thiol group and is present at the 5'end.
5'-GCGGCTCTGCGCATTCAATTGCTGCGCGCTGAAGCGCGGAAGCTTTTTTTTTTTT-3'
(SEQ ID NO: 3)

Preferably, the functional group for attaching the at least one aptamer to the metal electrode surface is a thiol group and might be present at the 3' end and/or 5' of SEQ ID NO: 3. In a preferred embodiment, the functional group for attaching the at least one aptamer to the metal electrode surface is a thiol group and is present at the 3'end.
Progesterone aptamer:

5'-GCATCACACACCGATACTCACCCGCCTGATTAACATTAGCC
CACCGCCCACCCCCGCTGC-3' (SEQ ID NO: 4)

Preferably, the functional group for attaching the at least one aptamer to the metal electrode surface is a thiol group and might be present at the 3' end and/or 5' of SEQ ID NO: 4. Preferably, the functional group for attaching the at least one aptamer to the metal electrode surface is a thiol group and is present at the 5'end.

Preferably, the invention relates to the biosensor device of the invention for use in diagnosis. Preferably, the biosensor device of the invention is for sensing a hormone. For example, the biosensor can be used for determining the nature of at least hormone and/or the concentration of the hormone. Sensing of hormones might be useful for monitoring hormones during assisted reproductive technology (ART) or during menopause or for monitoring the menstrual cycle. Sensing of hormones might also be useful to study the progression or therapy of a hormone disorder or to diagnose a hormone disorder such as polycystic ovary syndrome (PCOS) or endometriosis. Sensing of hormones might also be useful during hormone treatment during sex change, menopause and ART.

Preferably, the invention relates to the *in vitro* diagnosis, particularly for sensing at least one hormone. For example, *in vitro* diagnosis might be useful for diagnosing hormones from any sample derived from a subject, e.g., human or animal, including but not limited to a body fluid, particularly blood, serum, urine, interstitial fluid, or saliva. In vitro diagnosis might also be useful in basic research, e.g., for calibrating the biosensor (see also Examples herein).

Moreover, the invention relates to a method for operating a biosensor device according to any of the preceding claims, comprising the steps:
(a) contacting the biosensor membrane with a body fluid, particularly interstitial fluid;
(b) allowing the at least one aptamer of the biosensor membrane to bind the at least one hormone; and
(c) detecting the binding of the at least one hormone by electrically controlling the at least one metal electrode surface.

Preferably, step (b) is performed under physiological conditions.

Preferably, step (c) is performed by differential pulse voltammetry (DPV). DPV is an electrochemical technique used for signal detection and quantification in various applications. It involves applying a series of voltage pulses to an electrochemical cell and measuring the resulting current response.

Preferably, the biosensor device comprises at least one electrode comprising the metal electrode surface, which carries the biosensor membrane. Preferably, the biosensor device additionally comprises a working electrode, preferably a reference electrode, preferably a counter electrode Preferably, the biosensor device comprises an electronic control device for controlling the at least one metal electrode surface. Preferably, the electronic control device is configured, in particular programmed, to perform an electrical measurement of the at least one biological quantity by controlling the at least one meetal electrode surface. Preferably, the electrical measurement is a voltammetry, preferably a DPV (differential pulse voltammetry) measurement. DPV measurements will be explained below, in more detail.

The invention also relates to a method of using the biosensor device or implantable apparatus or system of the invention for the treatment of a hormone disorder in a subject, e.g., a human or an animal. Sensing of hormones in the context of a treatment might be useful during assisted reproductive technology (ART) or during menopause or for monitoring a disturbed menstrual cycle. Sensing of hormones might also be useful in the context of treating a hormone disorder such as polycystic ovary syndrome (PCOS) or endometriosis. Sensing of hormones might also be useful in the context of sex change. For example, the biosensor can be used for determining the nature of at least hormone and/or the concentration of the hormone and - depending on the outcome of the measurement - a hormone replacement therapy can be set up for the patient. In one embodiment, the method comprises the treatment of a hormone disorder, e.g., infertility, and comprises steps of:
(a) contacting the biosensing membrane with a body fluid of the subject,
   particularly interstitial fluid;
(b) allowing the at least one hormone to bind to the at least one aptamer of the biosensing membrane ;
(c) detecting the binding of the at least one hormone by electrically controlling the at least one metal electrode surface, particularly via voltammetry, e.g. DPV;
(d) determining the nature and/or concentration of the at least one hormone;
(e) treating the subject with progesterone in case the sensed progesterone level is ≥ a thresho.d level, preferably 1.5 ng/mL; and/or treating the subject with hCG in case the sensed estradiol level is within a range of about 1,000 to 2,000 pg/mL.

### Example 1

The following steps describe the method which has been used for preparing the aptamers prior to self-assembly coating of the electrode surface:
1) Spin down the lyophilized powder vial to reach the dried aptamer pellet (obtained as thiol-modified aptamers for estrodiol, LH and progesterone with a sequence as shown in SEQ ID NOs: 1 to 4 from Basepair bio company) in the vial.
2) Resuspension of aptamer pellet in 10 mM Tris, pH 7.5, 0.1 mM EDTA in nuclease-free water (0.6057 g Tris, 0.0184 g EDTA 500 ml DI water; Resuspension Buffer).
3) Add Resuspension Buffer to the aptamer pellet to reach 100 µM concentration.
4) Prepare Folding Buffer (1 mM MgCl₂, 1x PBS, pH 7.5) and dilute the aptamer solution to a 10x working concentration in the folding buffer. Heat the solution to 90-95 °C for 5 minutes.
5) Let the solution cool down to room temperature for 15 minutes.
6) Prepare a Reduction DTT buffer (20 mM DTT in 1x TE buffer (10 mM Tris, pH 8; 1 mM EDTA) or /20 mM in Tris-HCl buffer, 1 M Dithiothreitol (DTT). This step should be done immediately prior to use.
7) Reduce the thiol-modified aptamer using 20 mM DTT in 1x TE buffer, and use an equal volume of folded aptamer (1:1) with aptamer-reducing buffer and incubated at room temperature (20-25 °C) for 1 hour.
8) Remove the DTT using a desalting or buffer exchange column.
9) The aptamer are diluted to the final working concentration using a buffer containing 1 mM MgCl₂ (in nuclease free water PBS).
10) The aptamer is ready for coating of the electrode.

### Example 2

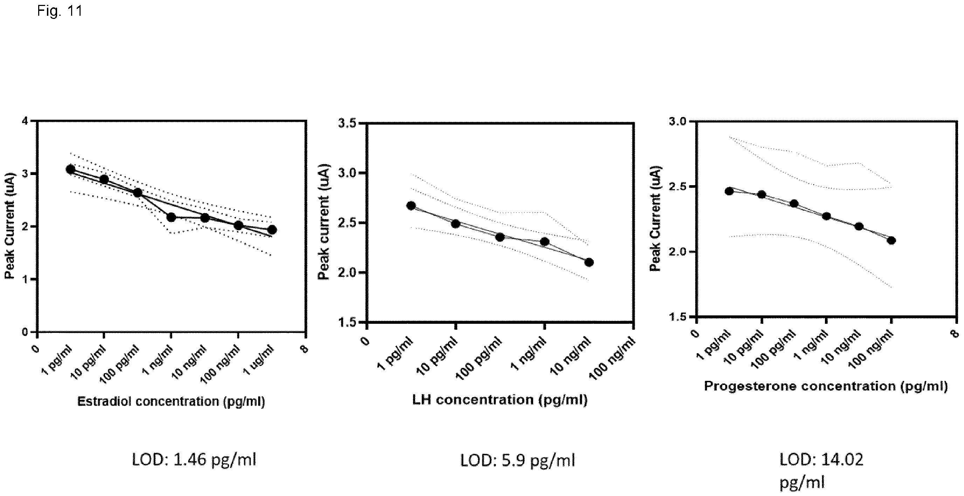

The following steps describe the method that has been used for preparing the functionalized gold electrodes for hormone measurement:
1) Simultaneous to the aptamer preparation (Example 1), the gold electrode on the glass is prepared.
2) The electrode is activated using three-electrodes (counter electrode, reference Ag/CI electrode, and gold working electrode) and cyclic voltammetry for 45 segments in 50 mM sulfuric acid.
3) Followed by this step, the sensor was washed with 10 mM sulfuric acid gently.
4) DPV (Differential Pulse Voltammetry) signal of the gold electrode was recorded before the addition of the aptamers.
5) 10 µl of aptamer solution was added to the gold working electrode to a concentration of 5 mM and incubated for 16 hours for immobilization of aptamers on gold electrode via self-assembled monolayer.
6) Afterwards, excess aptamers are washed from the surface using sterile PBS.
7) 2 mM MCH (6-Mercapto 1-Hexanol) is added to the electrode as the blocking agent and incubated for 1 hour.
8)The DPV signal is recorded for drop-casting of the hormone stock solutions in ISF (interstitial fluid) (from 1 pg/ml to 1 ug/ml).
9) Finally, the peak current of each DPV signal is analyzed to measure the sensitivity.
10) The experiments are repeated at least three times and results are shown in Figure 11.

According to a preferred aspect, the invention is related to an **implantable apparatus/ implantable biosensor device.**

Implantable biosensors involve a risk of rejection or adverse reactions due to various factors, including the body's immune response and the materials used in the implants.

When a foreign object is introduced into the body, the immune system recognizes it as non-self and may mount an immune response to eliminate or neutralize it. This immune response can manifest as inflammation, swelling, pain, or even an immune-mediated rejection of the implant. The severity of the immune response varies among individuals and can be influenced by factors such as genetics and overall health. There exists a need for reducing the risk of a severe immune response when using an implantable biosensor. Implantable devices are therefore made from materials that are biocompatible, meaning they are designed to minimize the adverse effects on living tissue. The choice of materials used in implants plays a crucial role in determining their compatibility with the body. Some materials may be more prone to triggering immune responses or causing adverse reactions than others. For instance, certain metals or polymers may induce hypersensitivity reactions or release toxic substances when in contact with bodily fluids or tissues.

Implantation procedures carry a risk of introducing bacteria or other microorganisms into the body, which can lead to infection. Infections can cause localized inflammation, tissue damage, and may even result in the need for implant removal. There exists a need to provide implantable biosensors having attributes for minimizing the risk of introducing bacteria or other microorganisms into the body.

The skill and technique of the surgeon performing the implantation procedure can also influence the risk of adverse reactions. Improper implant placement, trauma during surgery, or inadequate sterilization can increase the likelihood of complications and subsequent rejection. There exists a need to provide implantable biosensors which are easy to apply by a surgeon.

Implantable devices involve significant financial investments, including the cost of the device itself, surgical procedures, and post-implantation care. It is therefore important to maximizing the longevity of an implantable for helping to optimize the cost-effectiveness of the treatment. If an implantable has a longer lifespan, it reduces the need for frequent replacements or revisions, thereby reducing overall healthcare costs.

Implantable devices are often intended to provide long-term solutions for medical conditions or disabilities. A longer lifespan of the implantable translates to a longer period of improved functionality, convenience, and improved quality of life for the patient. Avoiding frequent replacements or maintenance procedures can minimize disruptions to daily life and reduce the burden on patients and caregivers.

Implantation procedures carry inherent risks, including potential complications such as infection, bleeding, and tissue damage. Minimizing the need for additional surgeries or revision procedures due to implant failure or degradation reduces the cumulative risks associated with multiple surgeries. It also decreases the potential for additional complications and their associated healthcare costs.

Implantables often play a vital role in monitoring and managing medical conditions. For example, implantable sensors for glucose monitoring in individuals with diabetes provide valuable data for treatment adjustments. Longevity of such devices ensures continuous and reliable data collection, allowing healthcare professionals to make informed decisions regarding treatment plans.

Implantables should be designed to maintain their functionality and biocompatibility throughout their intended lifespan to minimize the risk of complications, adverse reactions, or device failure. Regular monitoring, follow-up care, and periodic evaluations of implantable devices are necessary to ensure their continued efficacy and safety.

An object underlying the present invention according to an aspect of the invention is therefore to overcome one or more of the problems of implantable biosensors described above, in particular to provide an implantable apparatus, which has a sufficient longevity and provides long-term safety.

The object is solved by the implantable apparatus according to a preferred aspect of the invention, which is an implantable apparatus for sensing at least one biological quantity inside the body of an animal or a human, in particular for being implanted, in particular subcutaneously implanted, into the body of an animal or human, comprising a biosensor device for sensing a biological quantity in vivo and an electronic control device for controlling the biosensor device, wherein the implantable apparatus comprises an encapsulation device for at least partly encasing at least one of the biosensor device and the electronic control device and wherein the encapsulation device is at least partly made from glass.

The encapsulation device may be a single part device, or may comprise more than one encapsulation parts forming the encapsulation device. When using multiple encapsulation parts, assembly of the implantable apparatus may be facilitated. Preferably, the encapsulation device includes two encapsulation parts, which may together encase a cavity for receiving the electronic control device and preferably, other functional devices, e.g. a communication device or the biosensor device. Respectively preferably, the encapsulation device includes exactly one, two, three, four, five or six encapsulation parts, or more encapsulation parts, which may together encase said cavity. Preferably, at least on, two, three, four, five or more of said encapsulation parts comprise glass or are partly or completely made from glass, in particular substantially completely made from glass. The glass preferably is transparent, or preferably is opaque.

Preferably, the encapsulation device includes at least one wafer substrate comprising glass or are partly or completely made from glass, wherein "completely made from glass" includes the case that those wafers are in particular substantially completely made from glass, which means they may include non-glass portions in a low fraction. The glass preferably is transparent, in part or completely, and/or preferably is opaque, in part or completely. Preferably, the glass wafer includes a plurality of vias, which are open or preferably filled with a non-glass material, in particular a metal, which are in particular cylindrically shaped and may have a height of about 100 to 1000, preferably 200 to 800, preferably 300 to 700, preferably 400 to 600, preferably 500 micrometer, a diameter between preferably 20 to 500, preferably 40 to 200, preferably 50 to 150 micrometer, and a pitch preferably between 1000 and 20, preferably between 800 and 30, preferably between 500 and 50 or preferably between 300 and 50 micrometer. For example, HermeS^{®} glass wafer substrates, available from Schott AG, Germany, are glass wafers being substantially made from glass. They include hermetically sealed, solid through-glass vias (TGV). The fine-pitched vias enable reliable conduction of electrical signals and power into and out of the implantable apparatus using such glasses, because the vias are solidly filled with a conductive metal. A wafer substrate, in general, is understood to mean a solid and plain plate-like or disk-like element acting as a substrate for at least one electrode or other sensing element. Preferably, a wafer substrate is an element, which was cut out from a larger wafer, in particular glass wafer.

Preferably, the wafer substrate contains at least one metal filled via, which is connected to a device of the implantable apparatus, in particular to any of the following devices: electronic control device, biosensor device, in particular at least one electrode of the biosensor device, communication device. Preferably, the wafer substrate contains at least one metal filled via, which is connected to at least one sensing element, in particular at least one electrode, of the biosensor device, on one side of the wafer substrate and is connected to the electronic control device on the other side of the wafer substrate. Preferably, the wafer substrate contains at least one metal filled via, which is connected to the communication device on one side of the wafer substrate and is connected to the electronic control device on the other side of the wafer substrate.

A wafer substrate is understood to be a thin, substantially flat, and preferably substantially rectangular or substantially circular piece of material. The wafer substrate preferably provides a solid and uniform surface, on which, in particular, various electronic structures, circuits or electrodes can be built, which may form part of the electronic control device and/or the communication device.

The wafer substrates are preferably made from glass. They may also be built by another amorph or crystalline material, for example made from semiconductor materials such as silicon (Si). Glass wafers are preferred due to their desirable electrical and mechanical properties, availability, and compatibility with standard lithography processes. Moreover, glass provides excellent biocompatibility, even without a biocompatible coating, which, however, is also a preferred option for an encapsulation device or respectively, a wafer substrate, consisting partly or completely of glass or a non-glass material. The coating may be a glass coating.

The inventors of the present invention discovered that glass can be used to create safe and reliable encapsulation device, which contain, in particular, one ore more hermetically sealed cavities with long-term life cycle.

The glass of the encapsulation device may be, respectively preferably, any of a bioactive glass, borosilicate glass, aluminosilicate glass, phosphate glass, silica glass or chalcogenide glass. Bioactive glasses have the ability to bond with living tissues. They typically contain calcium, phosphorus, and silica as their main components. Borosilicate glasses, such as Pyrex, have a high chemical resistance and thermal stability. Aluminosilicate glasses are a family of glasses that contain aluminum and silicon. They have good mechanical strength. Phosphate glasses typically contain phosphorus pentoxide (P2O5) as a major component. They exhibit high solubility in aqueous solutions. Silica glasses, such as fused silica, are composed mainly of silicon dioxide (SiO2). They have good optical transparency, low thermal expansion, and good chemical stability. Chalcogenide glasses consist of elements from the chalcogen group, including sulfur, selenium, and tellurium. They have unique optical and electrical properties. Preferably, the glass is any of Borofloat^{®} 33, AF 32^{®} eco 33, or D 2630 T eco, all available from Schott AG, Germany. Preferably, the encapsulation device is made from one type of glass material, which allows for a facilitated assembly of the encapsulation device.

In a preferred aspect, the at least one wafer substrate carries one or more metal electrodes, which in particular, form a part of the biosensor device, and which, preferably carry a biosensor membrane.

In a preferred aspect, the at least one wafer substrate carries the electronic control device, which in particular, is used to control a measurement process using the biosensor device.

In a preferred aspect, the at least one wafer substrate carries the communication device, in particular an RFID device, in particular a NFC device.

In a preferred aspect, one wafer substrate carries two or all of the following devices: the electronic control device, at least one sensing element, in particular at least one electrode, of the biosensor device and the communication device.

In a preferred aspect, the encapsulation device includes two wafer substrates, which are stacked on top of each other, in particular arranged in parallel, and which are preferably arranged in distance for encasing a cavity, which in particular may contain one or more of the electronic control device, the biosensor device and the communication device. Said distance may be preferably between 200 and 5000, preferably between 300 and 3000, preferably between 500 and 2500 micrometer. The sides of the cavity may be closed by bar members, which may be integral with any of the wafer substrates or which may be separate parts, which are preferably joined, in particular by welding or gluing, to any of the substrate wafers.

In a preferred aspect, at least two glass parts, in particular two glass wafer substrates, of the encapsulation device of the implantable apparatus are preferably aligned in a stacking, and bonded through femtosecond laser stitching. This technique was surprisingly found to ensure complete hermeticity and in turn to protect the electronics and internal cavities from exposure to aforementioned physiological body fluids, even under stress conditions and during a long-term test use of the implantable apparatus.

In a preferred aspect, the encapsulation device includes a stacking of two, three, four or more wafer substrates, which are preferably arranged in a distance for encasing a cavity.

A wafer substrate may comprise a flange portion, which form a trough shaped substrate member. The hollow portion of such a trough may act as the cavity for receiving or covering any of the electronic control device, the biosensor device and the communication device.

The encapsulation device may have a capsule-form, when assembled. This facilitates the implantation of the implantable apparatus using a syringe, for example.

The encapsulation device may have a box-form, when assembled, and be in particular a cuboid, preferably a rectangular cuboid. The edges and/or corners, respectively, may be rounded, in particular having a rounding radius between preferably 0.05 and 5 mm, between preferably 0.1 and 3 mm.

In a preferred aspect, the encapsulation device includes a main wafer substrate, and a trough-shaped top lid wafer substrate. The top lid wafer substrate may cover one side of the main wafer substrate and define a cavity between the side of the main wafer substrate and the hollow space defined by the trough-shaped top lid wafer substrate.

Preferably, the encapsulation device includes a trough-shaped bottom lid wafer substrate. The bottom lid wafer substrate may cover another side of the main wafer substrate and define a cavity between the other side of the main wafer substrate and the hollow space defined by the trough-shaped bottom lid wafer substrate.

The encapsulation device, in particular at least one wafer substrate, in particular the at least one trough-shaped wafer substrate, may include one or more pores. The pores may be configured to allow an aqueous solution, in particular a body fluid, to enter the cavity covered by the at least one wafer substrate. A wafer substrate being suitable for forming such a porous surface is CoralPor^{®} porous glass, commercially available from Schott AG, Germany. The pore geometry may be cylindrical, the pore height may be between preferably 200 and 800 µm, between preferably 300 and 600 µm, between preferably 360 and 580 µm, the pore diameter may be preferably between 10 to 500 µm, preferably between 10 to 300 µm, preferably a pore size between 20 to 150 µm, preferably between 32-80 µm, preferably a porosity between 40 - 80 %, preferably between 60 and 70 %, preferably between 60 - 75 %.

Preferably, the encapsulation device fully encases the electronic control device, or the encapsulation device fully encases the electronic control device and the communication device, or the encapsulation device fully encases the electronic control device, the communication device and the biosensor device, wherein the full encasement is such that the respectively fully encased electronic control device and/or communication device is not in contact with body fluids when the implantable apparatus is implanted in a human or animal body, and/or, in particular, that the biosensor device is contactable with body fluids when the implantable apparatus is implanted in a human or animal body.

Generally, the encapsulation device may consist in part or completely of at least one non-glass material, for example, a polymer. In another example, the biosensor device may be encapsulated by embedding the same in a sealing compound, which may be biocompatible or may comprise a biocompatible coating layer.

The electronic control device, respectively preferably, include one or more or all of the following components: a microcontroller, in particular a low power or ultra-low power microcontroller; one or more integrated circuits (ICs), in particular an ASIC (application-specific integrated circuit; ASIC); one or more electronic amplifiers for amplifying the electric measurement signals, in particular currents, received from the biosensor device; a light source, preferably an LED, for visually sending information to a user of the implantable apparatus, or another output device for sending a signal to a user, for example an acoustical signal. The electronic control device may be part of the biosensor device or may be connected to the biosensor device.

An ASIC is a type of integrated circuit (IC) that is designed and fabricated to perform a specific set of functions within a particular application or system. Unlike general-purpose ICs, which are designed to perform a wide range of functions, ASICs are customized and optimized for a specific application, providing high performance, lower power consumption, and often reduced cost compared to other solutions. However, the integrated circuit may also be a general-purpose IC being programmed, for example, to fulfil the specific set of functions.

The specific set of functions may include: controlling the one or more electrodes of the biosensor device for performing a measurement process, in particular by measuring the current or impedance between at least two electrodes of the biosensor device as the potential applied to the at least two electrodes is varied. The measurement preferably is a DPV. The specific set of functions may include: receiving measurement signals from the biosensor device and derive therefrom measurement data. The specific set of functions may include: send the measurement data to an external data processing device (e.g. a mobile phone, tablet PC, PC) via the communication device. The specific set of functions may include: receiving electric energy from radio waves arriving at the implantable apparatus from outside, via the communication device, in particular the RFID device, in particular the NFC device, and, preferably, powering the measurement process using said electrical energy.

Preferably, the electronic control device is programmed to implement the specific set of functions.

DPV stands for Differential Pulse Voltammetry, which is an electrochemical technique used for signal detection and quantification in various applications. It involves applying a series of voltage pulses to an electrochemical cell and measuring the resulting current response. The preferred electrochemical cell setup for a DVP is as follows: A working electrode, a reference electrode, and a counter electrode are placed in an electrochemical cell. The DPV technique applies a series of voltage pulses to the working electrode. The pulses typically consist of a step potential, a holding potential, and a pulse width. The step potential provides the driving force for the electrochemical reaction, and the holding potential allows time for the system to reach equilibrium before the next pulse. Current measurement: After each voltage pulse, the resulting current response is measured using a potentiostat. The current response corresponds to the electrochemical reaction occurring at the working electrode. The obtained current responses are typically plotted against the applied voltage pulses. The resulting DPV curve exhibits characteristic peak shapes that can provide information about the analyte concentration, redox reactions, and other electrochemical processes occurring in the system with the analyte of interest, facilitating the electrochemical reaction.

The electronic control device may comprise at least one program code for implementing the specific set of functions, which may also contain one or more of the following functions; the electronic control device preferably is programmed to implement one or more of the following functions:
- Process the measurement data obtained by the biosensor device, the measurement data being derived from the measurement process executed by the biosensor device;
- Encrypt the measurement data using an encryption algorithm, in particular a lightweight encryption algorithm;
- establish a data connection between the communication apparatus of the at least one biosensor device, or respectively, the at least one implantable apparatus, and the communication device of the external computer device, e.g. by using a data communication protocol, for example a known communication protocol like TCP/IP, or a custom made communication protocol;

- Exchange data between the communication apparatus of the at least one biosensor device, or respectively, the at least one implantable apparatus, and the communication device of the external computer device, e.g. by using a data communication protocol, for example a known communication protocol like TCP/IP, or a custom made communication protocol;
- Storing the data received from the communication apparatus of the at least one biosensor device, or respectively, the at least one implantable apparatus, in a data storage, which may be part of the biosensor device or respectively, the implantable apparatus, or be connected to the same.

The communication device preferably is programmable device and is, in particular, programmed to receive measurement data from the electronic control device, preferably in an encrypted form, and to send the measurement data by wireless connection to an external computer device, in particular by RFID, preferably NFC, or WLAN.

RFID stands for Radio Frequency Identification, and an RFID device refers to a system or device that uses radio waves to wirelessly exchange data using radio frequency electromagnetic fields. An RFID system for data exchange typically consists of two main components: an RFID transponder and an RFID reader, which may be part of an external computer device, particularly a mobile phone. An RFID transponder is a small electronic device that contains a microchip and an antenna. The microchip stores measurement information /measurement data about generated by the biosensor device. The antenna enables communication with the RFID reader using radio waves. The RFID transponder is preferably passive, but can also preferably be active. Passive RFID transponders do not have an internal power source and rely on the energy transmitted by the RFID reader to power the RFID transponder. When the reader emits radio waves, the transponder's antenna captures the energy and uses it to activate the microchip, preferably to energize the measurement process, and transmit the stored measurement data back to the reader. Active RFID transponders have their own internal power source, usually a battery, which may be part of the implantable apparatus. This allows them to transmit signals continuously or on demand, providing a longer read range and the ability to store more data. Active transponders are typically used in applications that require longer-range or real-time tracking. An RFID reader is a device that emits radio waves and receives signals from RFID transponder. It consists of an antenna, a transceiver to send and receive radio signals, and a decoder to interpret the data received from the tags. The reader communicates with the tags wirelessly and can read multiple tags simultaneously within its range. When an RFID transponder comes into the range of an RFID reader, the reader's radio waves activate the tag. The transponder responds by sending its stored data back to the reader, which then decodes and processes the information. The reader can be connected to a computer or a backend system to further process and utilize the data collected from the transponders.

NFC stands for Near Field Communication, and an NFC device refers to a type of device that is equipped with NFC technology, allowing it to communicate wirelessly with other NFC-enabled devices or transponders within close proximity. NFC is a short-range wireless communication technology that operates at a frequency of 13.56 MHz. NFC devices typically include smartphones, tablets, smartwatches, and other electronic devices that have an NFC chip embedded within them. These devices can interact with each other by bringing them into close proximity or by tapping them together.

NFC enables contactless communication between devices. By simply placing or tapping two NFC-enabled devices together, they can establish a connection and exchange data. NFC allows for the transfer of various types of data, such measurement data, between devices. This data exchange can be initiated by a user or triggered by specific applications or services. NFC technology provides a convenient and secure method for wireless communication and data transfer over short distances.

The communication device preferably is an RFID device, preferably an NFC device.

The biosensor device includes at least one sensing element, in particular at least one electrode, which may carry a biosensor membrane, for example an aptamer containing membrane for measuring the binding, or respectively the surface concentration on the electrodes of biomarkers, preferably hormones, to the aptamers during a measurement process. The biosensor membrane is configured to be contacted by a body fluid for measuring the biological quantity within the body fluid. Preferably, the electrodes include a reference electrode an one or more working electrodes, wherein one working electrode carries said biosensor membrane. The biosensor device may be a stand-alone device. The biosensor device, preferably, may comprise the electronic control device and/or the communication apparatus. The biosensor device may be understood to include the same parts as the implantable apparatus, except from the encapsulation device. Without the encapsulation device, the biosensor device may be used as an in vitro solution or externally from the body of a human or an animal, for example in contact with the skin tissue of a body.

The biosensor device may be a passive device, which is powered by the energy from radio waves received via the communication apparatus for interrogating the biosensor device from an external computer device. The external computer device, for example a mobile phone, may comprise a communication apparatus capable to emit said radio waves for interrogating the biosensor device. Said communication apparatus, generally, is capable for a wireless data exchange with at least one biosensor device. In particular, the biosensor device and/or the implantable apparatus is a battery-less device/apparatus.

However, it is also preferred that the biosensor device comprises a battery for providing electrical energy for running the measurement process using the biosensor device, and/or for providing electrical energy for exchanging data with at least one external computer device. "External", in this context, means that the computer device is separate from the biosensor device and the implantable apparatus, respectively. The implantable apparatus is separate from the external computer device when the implantable apparatus is implanted into the body of a human or an animal.

The at least one sensing element may be an electrode. In this case the at least one biological quantity is measured using an electrical measurement process, in particular a voltammetry measurement or a capacitive measurement.

The at least one sensing element may be an optical sensing element, which may comprise a radiation source, in particular light source, and/or a radiation sensor, for example a photodetector. In this case the at least one biological quantity is measured using an optical measurement process, in particular a measurement sensible for alterations of a refractive index of a test material, or sensible for alterations of an amplitude and/or radiation spectrum (light spectrum) of a sensing radiation (light) emitted by a radiation source. The optical sensing element may be an optochemical sensing element, which is capable to detect the presence or alterations of a concentration of at least one chemical or biological compound being attached to a measurement surface of the optochemical sensing element, the chemical or biological compound originating from placing the measurement surface in contact with a fluid, in particular body fluid, to be measured.

The at least one electrode may be from gold. It may additionally carry at least one or more layers of metallic nanoparticles, in particular gold nanoparticles, which are commercially available. Thereby, the measurement surface of the biosensor membrane is increased - the surface may then be not planar but surrounding the nanoparticles. A layer of platinum nanoparticles may be provided on the layer of gold nanoparticles. It was surprisingly found by the inventors that a combined layer of gold and platinum nanoparticles efficiently increases the longevity of the biosensor device, and therefore, of the implantable apparatus. The gold nanoparticles are preferably attached to the one or more electrodes, in particular working electrodes. The platinum nanoparticles are preferably attached to layer of gold nanoparticles.

The invention is also related to a system for sensing at least one biological quantity inside the body of an animal or a human, the system comprising:
- at least one biosensor device according to the invention, or respectively, at least one implantable apparatus according to the invention
- at least one external computer device being separate from the at least one biosensor device, or respectively, the at least one implantable apparatus, the at least one external computer device including a communication device, in particular an RFID, NFC or WLAN device for wireless exchange of data, in particular measurement data, with a communication apparatus of the at least one biosensor device, or respectively, the at least one implantable apparatus.

The system may also include a program code to be executed by the external computer device, which is configured to implement a data exchange between the communication apparatus of the at least one biosensor device, or respectively, the at least one implantable apparatus, and the communication device of the external computer device. In particular, the external computer device may be programmed, in particular by using said program code, to implement one or more of the following functions:
- establish a data connection between the communication apparatus of the at least one biosensor device, or respectively, the at least one implantable apparatus, and the communication device of the external computer device, e.g. by using a data communication protocol, for example a known communication protocol like TCP/IP, or a custom made communication protocol;
- Exchange data between the communication apparatus of the at least one biosensor device, or respectively, the at least one implantable apparatus, and the communication device of the external computer device, e.g. by using a data communication protocol, for example a known communication protocol like TCP/IP, or a custom made communication protocol;
- Decrypting the encrypted data, in particular encrypted measurement data received from the communication apparatus of the at least one biosensor device, or respectively, the at least one implantable apparatus;
- Processing the data, in particular the decrypted data, in particular evaluating the data for obtaining measurement values form said data;
- Storing the data received from the communication apparatus of the at least one biosensor device, or respectively, the at least one implantable apparatus, in a data storage, which may be part of the external computer device or be connected to the same.

It is noted that the method of preparing gold electrodes on a glass wafer substrate is an invention, independently from the other inventive aspects of the implantable apparatus and the biosensor device. The method comprises the following steps:
- Providing a glass wafer substrate;
- Depositing an electrode structure made of an adhesion promoting layer for improving the gold adhesion on the glass wafer substrate, the adhesion promoting layer being preferably made from NiCr or Chrome, in particular by using vapor deposition with a NiCr or Ni Source, in particular by implementing a lithographical process:
- Depositing the gold on the electrode structure, in particular using vapor deposition with gold atoms.

Preferably the method for preparing gold electrode on a glass wafer comprises any of the following steps:
- clean the glass wafer substrate from any contaminants.
- Use a suitable photoresist to create a pattern for the electrodes on the glass wafer substrate.
- Create a photomask with the desired electrode pattern. This mask will be used to expose the photoresist and create the desired pattern.
- Prepare the developer solution according to the manufacturer's instructions.
- Use a suitable NiCr, or Ni, or gold source for vapor deposition, such as a (gold) evaporator.
- Immerse the glass wafer substrate in a cleaning solution to remove surface contaminants.
- Rinse the glass wafer substrate thoroughly with distilled water and let it dry.
- Apply the photoresist evenly onto the surface of the glass wafer substrate.
- Use a spin coater or other suitable method to ensure uniform coverage and a smooth surface.
- Allow the photoresist to dry according to the manufacturer's instructions.
- Place the photomask over the glass wafer substrate.
- Expose the glass wafer substrate to UV light according to the manufacturer's instructions for the photoresist.
- The exposure will cause the photoresist to become soluble in the exposed areas and remain insoluble in the masked areas.
- Immerse the exposed cover glass in the developer solution following the manufacturer's instructions.
- The developer will remove the soluble photoresist, revealing the electrode pattern.
- Place the prepared glass wafer substrate with the exposed electrode pattern in a vacuum chamber together with the NiCr, or Ni, or gold source.
- Create a vacuum in the chamber and evaporate the gold from the NiCr, or Ni, or gold source.
- The NiCr, or Ni, or gold atoms will condense onto the surface of the glass wafer substrate, forming a thin NiCr, or Ni, or gold layer that covers the electrode pattern.
- Depending on the specific requirements, one may need to perform post-treatment steps such as annealing or surface modification to enhance the adhesion and properties of the NiCr, or Ni, or gold electrodes.

By following these steps, one can lithographically apply NiCr, or Ni, or gold electrodes to a glass wafer substrate using vapor deposition with gold atoms.

Each electrode has a complex layer structure to ensure that there is sufficient electrical contact to the biosensor. Each layer contributes the performance of the single-atomic-layer that binds to the biosensor to generate electronic signals. The nano-layer is deposited on the wafer to maximise electronic contact with the aforementioned biosensor. This process is deposited through atomic-layer deposition methods to achieve critical contact with the adhesion layer.

Mask/etching preparation of the back/external side of the wafer, in a preferred fabrication step: a biocompatible glass wafer of cut diameter ~100mm (Can be up to 300mm) is prepared and cleaned in a preparation solution to improve adhesion of the first deposited layer.

The wafer is prepared with a positive mask and a negative mask during the in-process steps to allow for the thin film metallised layer to be applied. A glass adhesion layer is advantageously used to bond and provide an appropriate tensile strength (50N) to allow for additional layers to be metallized on the prepared surface.

The wafer is now ready for either additional metallisation to increase the layer height to 50 000 nm using electroplating or activation of the Au surface to allow for the biosensor to be attached to the electrode.

A biosensor device may be prepared in three stages,
1) Activation of the deposited gold layer on glass, this stage is done by connecting the working electrode (gold) to potentiostat and the continuous cyclic voltammetry (CV) in 50 mM sulfuric acid. While the CV is done the electrode is cleaned with ethanol and DI water.
2) Deposition of gold nanoparticles on the gold layer on top of the glass. This stage is done to increase the surface area of the gold surface. And finally:
3) activation of the biosensor membrane, the biosensor membrane comprising of a self-assembled monolayer (SAM) of aptamers, the SAM layer being responsible for the permanent attachment of the aptamer to the gold platform.

The biosensor membrane may contain molecules for binding a biomarker from the body fluid. The molecules may be aptamers, or respectively, antibodies. The hormones preferably being any of Estradiol, LH, Progesteron.

The invention is also defined by a method for operating an implantable apparatus according to the invention, or a biosensor device, comprising the steps:
a. Contacting the biosensor membrane with a body fluid;
b. Allowing biomarkers, in particular hormones, to bind to the biosensor membrane;
c. Detecting the binding of hormones to the biosensor membrane by electrically controlling the at least one metal electrode surface.

The invention is related, in particular, to an implantable apparatus for being implanted, in particular subcutaneously implanted, into the body of an animal or human, comprising a biosensor device, in particular a biosensor device according to the claims, for sensing a biological quantity in vivo and an electronic control device for controlling the biosensor device, and is related to the following, respectively preferred aspects of said implantable apparatus, wherein the implantable apparatus according to each aspect may be claimed by a patent claim or wherein the implantable apparatus according to a combination of multiple aspects may be claimed by a patent claim:
According to aspect 1, the implantable apparatus comprises
   an encapsulation device, which is described herein in many aspects, in particular for at least partly encasing at least one of the biosensor device and the electronic control device, wherein the encapsulation device is at least partly made from glass.
According to an aspect 2, the implantable apparatus, which is in particular configured according to aspect 1, further comprises a communication device, in particular NFC device, connected to the electronic control device for a wireless data communication with an external device.
According to aspect 3, the implantable apparatus, which is in particular configured according to aspect 1 or aspect, 2 is further defined by
   wherein the encapsulation device fully encases the electronic control device, or
   wherein the encapsulation device fully encases the electronic control device and the communication device, or
   wherein the encapsulation device fully encases the electronic control device, the communication device and the biosensor device,
   wherein the full encasement is such that the respectively fully encased electronic control device and/or communication device is not in contact with body fluids when the implantable apparatus is implanted in a human or animal body, and/or, in particular, that the biosensor device is contactable with body fluids when the implantable apparatus is implanted in a human or animal body.
According to aspect 4, the implantable apparatus, which is in particular configured according to aspect 1 and, preferably, according to any of the aspects 2 to 3, is further defined by wherein the encapsulation device comprises at least one wafer substrate, which is substantially made from glass.
According to aspect 5, the implantable apparatus, which is in particular configured according to aspect 4 and, preferably, according to any of the aspects 2 to 3, is further defined by
   wherein the encapsulation device comprises at least one wafer substrate, which carries one or more electrodes on one side of the wafer substrate, and which preferably carries at least one electronic part, in particular the electronic control device, on the other side of the wafer substrate, wherein, preferably, the one or more electrodes are connected to the at least one electronic part.
The invention is related, in particular, to a method for operating an implantable apparatus, or respectively, its biosensor device, according to any of aspects 1 to 5, comprising the steps:
   a. Contacting a biosensor membrane of the biosensor device with a body fluid;
   b. Allowing biomarkers, preferably hormones, to bind to the biosensor membrane;
   c. Detecting the binding or the surface concentration of hormones on the biosensor membrane by electrically controlling the at least one metal electrode surface.
According to aspect 6, the invention is directed to a system for sensing at least one biological quantity inside the body of an animal or a human, the system comprising:
   - at least one biosensor device according to the invention, or respectively, at least one implantable apparatus according to any of aspects 1 to 5, or any other embodiment described herein;
   - at least one external computer device being separate from the at least one biosensor device, or respectively, the at least one implantable apparatus, the at least one external computer device including a communication device, in particular an RFID, NFC or WLAN device for wireless exchange of data, in particular measurement data, with a communication apparatus of the at least one biosensor device, or respectively, the at least one implantable apparatus.
According to aspect 7, the invention is directed to a program code to be executed by an external computer device, which is configured to implement a data exchange between the communication apparatus of the at least one biosensor device, or respectively, the at least one implantable apparatus, and the communication device of the external computer device. In particular, the external computer device may be programmed, in particular by using said program code, to implement one or more of the following functions:
   - establish a data connection between the communication apparatus of the at least one biosensor device, or respectively, the at least one implantable apparatus, and the communication device of the external computer device, e.g. by using a data communication protocol, for example a known communication protocol like TCP/IP, or a custom made communication protocol;
   - Exchange data between the communication apparatus of the at least one biosensor de-vice, or respectively, the at least one implantable apparatus, and the communication de-vice of the external computer device, e.g. by using a data communication protocol, for example a known communication protocol like TCP/IP, or a custom made communication protocol;
   - Decrypting the encrypted data, in particular encrypted measurement data received from the communication apparatus of the at least one biosensor device, or respectively, the at least one implantable apparatus;
   - Processing the data, in particular the decrypted data, in particular evaluating the data for obtaining measurement values from said data;
   - Storing the data received from the communication apparatus of the at least one biosensor device, or respectively, the at least one implantable apparatus, in a data storage, which may be part of the external computer device or be connected to the same.
According to aspect 8, the invention is directed to a method of fabricating the biosensor device and/or the implantable apparatus according to the invention, comprising the steps:
   - Preparing at least one metal electrode surface, in particular electrode, preferably made from gold or comprising gold, on at least one surface of at least one wafer substrate, which preferably is a glass wafer substrate;
   - Preparing the biosensor membrane on the at least one metal electrode surface;
   - Optionally: arrange an electronic control device on the at least one wafer substrate;
   - Optionally: fabricating a encapsulation device using the at least one wafer substrate, for encapsulating a cavity of the biosensor device, or respectively, the implantable device.
According to aspect 8, the method according to aspect 8 comprises the steps:
   - Providing multiple wafers (two or more) containing a plurality of wafer substrates, for fabricating multiple implantable apparatuses in parallel, wherein the multiple wafers are preferably aligned in a stack;
   - Welding of the plurality of wafer substrates, in particular welding of the multiple wafers, at stitching points or areas, preferably for creating at least one cavity, such the resulting stack of welded glass wafer substrates, in particular the at least one cavity, encloses at least one of the components of the biosensor device or respectively, the implantable apparatus: electrodes, electronic control device, in particular microcontroller, amplifier(s), RFID (preferably: NFC) chipset; antenna
   - Optionally: cutting at least one or more of stacked and welded wafer substrates, which in particular carry and/or enclose one or more of said components, from the stacked and welded wafers.

Implantable sensor apparatuses currently available on the market rely on batteries to **power their operation** for a limited period of time, after which batteries need to be replaced or wirelessly charged. In either case, the drawbacks are multiple for the health and wellbeing of the patient. In the case of battery removal due to the need of performing extraplantation surgery to remove the batteries with all the associated health risks involved and potential surgical site infection. In the case of charging the battery due to the time required for battery recharge, which can inconveniently restrict patient's movements or body posture during wireless power transference.

It is therefore an object of the invention according this particular aspect of the invention to provide an biosensor device or respectively implantable apparatus, which avoids the disadvantages above described, such as for example long charging times for recharging of the device or extraplantation procedures.

The problem is solved by the biosensor device according to realization 1 and the method according to realization 15. Further embodiments are described in addition by realizations 2 to 14 and 16 and further preferred configurations described by the entire patent application documents. Any of the mentioned realizations of the biosensor device may be used alone or in combination with one or more of the further realizations, and also any aspects according to the invention, as disclosed herein.
The biosensor device according to a realization 1 of the invention is configured to be implanted inside the human or animal body, in particular to be subcutaneously implanted. Such a biosensor device configured to be implanted is also referred to as "implantable apparatus" and may include an encapsulation device for at least partly encasing at least one of the biosensor device and the electronic control device. According to realization 1, the biosensor device (1i) or implantable apparatus (1i) comprises at least one metal electrode surface, preferably part of an electrode, in particular an electrochemical sensing electrode, for detecting voltage equivalent data quantifying a biological quantity, and
an electronic control device (2i),
wherein the electronic control device (2i) comprises
   - at least one amplifier (3i), and
   - a microcontroller (5i) for digitization of the voltage equivalent data into digitized measurement data, and
   - a communication device (6i), in particular an NFC device, whereas the NFC device (6ii) is configured to transmit the digitized measurement data of the electrochemical sensor to an external device (28i) (uplink) and to receive specific data (100i) from the external device (128) (downlink), and further configured to be powered up by the external computer device (108), such that the biosensor device (101) is fully operational.

Preferably, the microcontroller (5i) is programmed to encrypt the digitized data into encrypted data and wherein the encrypted data is sent to the (near field) communication device (6i) and wherein the encrypted data is further transmitted to the external computer device (8i) by the near filed antenna (7i).

Preferably, the biosensor device comprises an electrochemical sensor for sensing at least one biological / biochemical analyte (a biological quantity) and an electronic circuit, for use in monitoring body hormone levels, in particular hormone levels related to fertility circles in women or during in vitro fertilization, wherein the electronic circuit comprises at least one amplifier (4i) to convert and amplify a current derived from the analyte into voltage equivalent data with respect to a reference voltage level, and at least one amplifier to generate the reference voltage level, and an, preferably ultra-low powered, microcontroller (5i) for digitization of the voltage equivalent data into digitized data, and a, preferably near field (NFC), communication device comprising a (near field) antenna, whereas the communication device (NFC device) is configured to transmit the digitized data of the electrochemical sensor to an external computer device (8i; uplink) and to receive specific data from the external computer device (downlink), and further preferably configured to be powered up by the external computer device, such that the biosensor device is fully operational, wherein the microcontroller (5i) is preferably programmed to encrypt the digitized data into encrypted data and wherein the encrypted data is sent to the communication device and wherein the encrypted data is further transmitted to the external computer device by the (near field) antenna (7i).

In the present context, an electrochemical sensor is understood as a device that measures and detects chemical substances by converting the chemical reactions occurring at an electrode into electrical signals. It utilizes the principles of electrochemistry to sense and quantify various analytes or target substances in a sample, i.e., at the site of implantation in the human or animal body. A biosensor device according to the invention preferably generally includes an electrochemical sensor, which includes the at least one electrode or respectively the at least on metal electrode surface.

To measure the analyte concentration, which is also referred to as to measure the analyte or for sensing the analyte, the sensor preferably includes a reference electrode. The reference electrode provides a stable electrical potential against which the analyte reaction is compared, ensuring accurate measurements. By monitoring the electrical current or potential changes resulting from the analyte reaction, the sensor can determine, in particular participate in determining, the concentration of the target substance in the sample.

In the present context a biological quantity or biological/biochemical analyte is understood as for example a hormone of the human or animal body, in particular hormone levels related to fertility circles in women or during in vitro fertilization.

In the present context an amplifier to convert and amplify a current derived from the analyte into voltage equivalent data is understood as a current to voltage converter, for example a transimpedance amplifier, which almost exclusively is implemented with one or more operational amplifiers. The voltage equivalent data corresponds to voltage equivalent levels of data generated by means of resistors.

The electrochemical sensor is connected to the electronic circuit via working electrodes. Therefore, the electronic circuit comprises at least one working electrode for electrically connecting the electrochemical sensor to the electronic circuit, in particular for connecting to the amplifier, in particular to the transimpedance amplifier, for converting the current supplied by the electrochemical sensor into a voltage, i.e. into a voltage equivalent, also called voltage equivalent data in accordance with the current data as data of the electrochemical sensor.

In the present context a reference voltage corresponds to a voltage reference level generated by an operational amplifier in combination with a voltage divider circuit topology followed by signal buffering by the amplifier.

In the present context ultra-low powered microcontroller is understood as a microcontroller which allows data to be processed with the smallest amount of system power needed.

In the present context a near filed communication device (NFC) is understood as an NFC chip tag, also known as an NFC tag or simply an NFC chip, which is a small electronic device that contains an integrated circuit (IC) with an NFC antenna, i.e. a nearfield antenna. The tag is designed to store and transmit data wirelessly using NFC technology. The NFC chip tag is understood as a passive device, meaning it does not have its own power source. Instead, it relies on the power provided by, e.g., the external device. When the external device comes into close proximity to the NFC tag, in particular within a few centimeters, the external device generates an electromagnetic field that induces a small current in the tag's nearfield antenna. The current powers the NFC chip, allowing it to communicate with the external device.

The NFC chip tag can be embedded or attached to a printed circuit board and/or to a flexible circuit board. The data stored in the NFC tag can range from simple text or URLs to more complex information, such as biosensor device details, product information, or commands for a specific action.

The NFC chip tag is versatile and can be programmed and reprogrammed multiple times, allowing for flexibility in its use. It can be read and written by NFC-enabled devices such as smartphones, tablets, or dedicated NFC readers. By tapping or bringing an NFC-enabled device, e.g., the external device, which can be a mobile device, close to the NFC tag, users can read the information stored in the tag or initiate specific actions based on the tag's programming.

The biosensor device is fully operational when it is able to convert a current from the electrochemical sensor to the operational amplifier into a corresponding voltage and pass it to the ultra-low powered microcontroller to digitize and encrypts it, send it to the NFC device, and transmit it from the NFC device to the external device, which decrypts the transmitted encrypted data. The biosensor device is not fully operational when the device is merely powered up by an external device at the NFC frequency, however without authorization, which for example allows the decryption.

The ultra-low powered microcontroller is programmed to encrypt the digitized data into encrypted data and wherein the encrypted data is sent to the near field communication device and wherein the encrypted data is further transmitted to the external device.

For sending the encrypted data of the microcontroller to the NFC device, an Inter-Integrated Circuit (I2C) interface is used. The I2C interface is a widely used serial communication protocol that allows multiple devices to communicate with each other over a shared bus. Although I2C and NFC are distinct protocols, the I2C protocol is combined with the NFC protocol. For example, an NFC-enabled device can be connected to a microcontroller or other peripheral using the I2C interface. This allows the NFC device to communicate with the microcontroller, which can then control other components or perform additional processing based on the NFC data. In such a setup, the NFC device would handle the NFC-specific communication, while the I2C interface would facilitate the communication between the NFC device and the microcontroller. In the present case, the microcontroller further comprises an encryption algorithm and is thus programmed to encrypt the digitized data provided. The encrypted data is sent via the I2C interface to the NFC. The feature has the effect, that the ultra-low powered microcontroller will not start any operation until receiving a valid command sequence from an authorized external device, e.g., an NFC enabled mobile phone running a respective application, whilst the power to generate the voltage level needed for operating the biosensor device, i.e. the harvested voltage level, can be generated by any NFC-enabled external device or by any external device with the same Radio Frequency (RF) as the frequency of the NFC without authorization.

Although the I2C communication protocol specifications for the transmission baudrate and byte format are common values, the data packet, i.e. the byte stream exchanged between the ultra-low powered microcontroller and the NFC chip tag is custom-made, such that, for example some of the transmission bytes that the microcontroller needs to perform, for example acquiring of the voltage equivalent data and data transmission to the NFC or control parameters for the operation of the biosensor device itself are encoded. Preferably, the byte stream or data packet generated for NFC transmission comprises byte fields occupied by the unique ID number of the implantable device plus the encrypted data obtained from the biosensors/temperature channels, control parameters and the security cypher for data decoding, totaling a total data payload of 1 kB in every NFC transmission (downlink/uplink).

Because the biosensor device is preferably batteryless, it does not suffer from the above shortcomings, remaining inside the human body, i.e. subcutaneously, for periods of time not dictated by a stored energy level, requiring only the presence of a nearby mobile phone with NFC capability for every cycle of device operation. This is not so inconvenient from the point of view of the patient because, for the intended application of the device, i.e., the monitoring of body hormones, readings can be taken sporadically and not in a continuous fashion way, thus diluting the impact of this reading procedure during the normal daylife routines of patients. Moreover, the implantable electronic circuit in here described does not actively stimulate the body tissues underneath by any means of physical transduction mechanisms, which could be electrical, light or ultrasounds, as other commercial devices, which makes it safer for use inside the body and acceptable to pass through the regulatory agencies around the world.

In a preferred realization 2, the external computer device is a NFC-enabled portable PC, tablet PC or mobile phone and the external computer device is programmed to decrypt the transferred encrypted data into decrypted data. The feature has the effect that no other mobile device is authorized to interact with the biosensor device. Furthermore, it makes reading out the data particularly easy for the patient, since the patient regularly carries his mobile device with him and thus no additional device is necessary. Furthermore, the data can be stored on the mobile device and easily sent to a doctor for evaluation, for example.

In a preferred realization 3 according to the invention the decrypted data is visualized on a display, in particular touch screen, of the external computer device.

The visualization of the transmitted data allows the patient to quickly obtain an overview of relevant hormone concentrations, for example, without having to have additional technical equipment.

In a preferred realization 4 according to the invention the external device is positioned less than 2 cm, or 2 cm, away from the biosensor device for the transmission of data, i.e. to establish up- or downlink. Such a distance is suitable for a subcutaneously implanted biosensor device, in particular. Both uplink/downlink and powering lines may operate up to a maximal gap distance of 2 cm between the implantable device and external communication device, in particular NFC source, without affecting the electronic performance metrics set for the implantable device (namely, nominal harvested voltage level and transmission quality).

In a preferred realization 5 according to the invention the sampling rate for digitization comprises a rate of 24 samples per second and a resolution of 10 bit or occurs at a rate of 24 samples per second and at a resolution of 10 bit. Such values are useful, because the resolution in terms of number of bits for the data collected from the biosensors/temperature then will set the limit of accuracy of the implantable device, whereas the number of samples per second will influence the size of the data packet (payload) for every NFC transmission, which is preferably set at a limit of 1 kB.

In a preferred realization 6 according to the invention the digitized data is temporarily stored in the micro controller. In order to perform data encoding, both data and intermediary results originated from the logic encryption operations (XOR) need to be temporarily stored inside the limited RAM memory of the microcontroller, prior to the inclusion of the processed encrypted data on the NFC data packet for transmission.

In a preferred realization 7 according to the invention the reference voltage level is generated during sensing of the at least one biochemical analyte. This feature enables higher measurement accuracy, as it allows changes in the reference voltage level, for whatever reason, to be taken into account. By fine tuning the reference voltage level, different chemical processes are set in motion that are more related to the target analyte (i.e., selection of a particular chemical functional group and/or improved geometrical alignment of specific molecular structures), thereby obtaining better accuracy for the measured signals, and this without the need for increasing the resolution (number of bits) of the digitization electronics.

In a preferred realization 8 according to the invention the electronic circuit comprises at least three working electrodes configured for sensing at least three analytes, in particular different analytes, of the electrochemical sensor. In further preferred realization of the invention the electronic sensor comprises two, four, five or even more working electrodes configured for sensing one, two, three, four, five, or even more analytes. This advantageously allows for measuring the same analyte with several working electrodes to increase accuracy of the biosensor device.

In a preferred realization 9 according to the invention sensing of the chemical analytes occurs simultaneously, such that the simultaneously generated currents provided by the electrochemical sensor are processed in parallel by the electronic circuit. The feature advantageously allows to monitor each of the different analyte concentrations simultaneously, for example by visualizing them on a mobile phone's display.

In a preferred realization 10 according to the invention the digitized data further comprises the temperature of the biosensor device. Providing information about temperature levels through the uplink communication channel as the microcontroller advantageously comprises an internal temperature indicator built-in on the chip die, for example allows further or more detailed analysis of the provided patient specific data.

In a preferred realization 11 according to the invention, the specific data may include user parameters comprising data for setting up ID numbers of the biosensor device, operating states of the biosensor device or control data of the sensing process of the biosensor device. The feature allows further personalization of the biosensor device, in particular to a patient-specific implant sensor.

In a preferred realization 12 according to the invention the biosensor device comprises an optical indicator, in particular a light emitting diode, configured to optically indicate the operating states of the biosensor device to a user. For example, a small, embedded light emitting diode (LED) gives visual feedback of the operations occurring at the implantable side when successfully powered up by the NFC interface of the mobile phone. This makes it easier to troubleshoot the biosensor device without having to remove an implanted biosensor device, for example.

In a preferred realization 13 according to the invention, the biosensor device comprises a rigid and/or a flexible printed circuit board (PCB). In particular, a flexible board can be used to avoid mechanical stress in the structure of the biosensor device and/or to achieve a specific, advantageous shape of the biosensor device. However, components may be also directly mounted on a wafer substrate, in particular without using a PCB.

In a preferred realization 14 according to the invention the (near field) antenna is wrapped in a number N of loops around the rigid PCB, wherein N is chosen preferably from the range 15 to 25 times, preferably 18 to 22, or N=20, and/or wherein the antenna comprises enamelled copper with a thickness of 0.15mm The internal NFC antenna allows that the AC radiofrequency waves generated by, e.g. the mobile phone are continuously captured by the NFC antenna, whereas the NFC antenna is wrapped around the external limits of the circuit board and converted to an equivalent DC level, referred to as the harvest voltage level, by the NFC chip tag itself, before making it available to the rest of the embedded electronics.

The problem is further, in a realization 15 of the invention, solved by the method of monitoring analytes, in particular body hormone levels inside a human or animal body, further particular hormone levels related to fertility circles in women or during in vitro fertilization, the method comprising the steps of:
- providing a biosensor device, including preferably an electrochemical sensor, for sensing at least one chemical analyte; and
- providing an electronic control device, in particular an electronic circuit, wherein the electronic control device comprises:
   at least one amplifier, in particular configured to convert and amplify a current derived from the analyte into a voltage equivalent data with respect to a reference voltage level, and
   preferably: at least one amplifier to generate the reference voltage level, and
   an, preferably ultra-low powered, microcontroller for digitization of the voltage equivalent data into digitized data, and
   a communication device, in particular a near filed communication device (NFC) comprising a -e.g. near field- antenna, whereas the communication device is configured to transmit the digitized data of the biosensor device or electrochemical sensor to an external computer device (uplink) and to receive specific data from the external computer device (downlink), and further preferably configured to be powered up by the external computer device, such that the biosensor device is fully operational, wherein
   preferably the microcontroller is programmed to encrypt the digitized data into encrypted data and wherein the encrypted data is sent to the communication device, e.g. the NFC device and wherein the encrypted data is further transmitted to the external computer device.
- Optionally: connecting the electrochemical sensor with the electronic sensor means at least one working electrode; and
- Preferably: providing energy to operate the biosensor device by means of the external computer device preferably using the wireless connection of thee devices, in particular using radio waves; and
- optionally visualizing of the transferred and decrypted data on a display of the external computer device.

In a preferred realization 16 of the biosensor device, in particular of the electronic control device, in particular the electronic circuit, the acquisition and amplification of ionic currents is produced by a total of 3 chemical analytes (or species) in simultaneous and contained within physiological solutions and/or fluids followed by digitization inside an ultra-low powered microcontroller, with final transference of the collected digital samples occurring through Near Field Communication (NFC) interface to an external mobile phone located at some distance, preferably less than 2 cm, from the electronic circuit. The same phone is responsible for delivering the necessary power to the implantable electronics (energy harvesting) through the radiofrequency field generated by NFC (13.56 MHz = Standard), which will enable electronics to reach fully operation to execute the aforementioned tasks of electrochemical acquisition as it does not rely in any form of stored internal energy or batteries. The implantable electronic circuit is intended to be used for monitoring body hormone levels directly related with the fertility cycle in women under normal physiological conditions or during in vitro fertilization (IVF) treatment.

Amperometric measurements refer to a type of electrochemical analysis that involves the measurement of electric current flowing through a system. Specifically, it involves the detection and quantification of analytes or chemical species of interest, based on the changes in current resulting from their electrochemical reactions at an electrode. In amperometric measurements, an electrode is immersed in an electrolyte solution containing the analyte. A potential is applied to the electrode, which initiates an electrochemical reaction involving the analyte. As the reaction proceeds, electrons are transferred between the electrode and the analyte, resulting in a measurable electric current. This current is directly proportional to the concentration of the analyte in the solution.

Further preferred configurations of the implantable apparatus, the biosensor device and systems according to the invention and the methods according to the invention emerge from the following description of the exemplary embodiments in conjunction with the **figures and the description** thereof. If nothing else is described or if nothing else emerges from the context, the same components of the exemplary embodiments are substantially characterized by the same reference signs. In detail:
Fig. 1 shows a schematic cross sectional view of an implantable apparatus according to an embodiment of the invention.
Fig. 2a shows a schematic perspective view of the implantable apparatus of Fig. 1.
Fig. 2b shows a top view of the top surface of an electrode carrying wafer substrate of an implantable apparatus according to an embodiment of the invention.
Fig. 2c shows a top view of the top surface of another electrode carrying wafer substrate of an implantable apparatus according to another embodiment of the invention.
Fig. 3 shows a top view of a section of a round wafer containing multiple electrode assemblies for cutting the same out from the wafer, for fabricating multiple electrode carrying wafer substrates as shown in Fig. 2b, each for an implantable apparatuses according to an embodiment of the invention.
Fig. 4a shows a perspective side view of an implantable apparatus according to an embodiment of the invention being made of a top wafer substrate, a medial wafer substrate and a bottom wafer substrate.
Fig. 4b shows a perspective side view of the medial wafer substrate of the implantable apparatus according to Fig. 4a, wherein the medial wafer substrate is shown upside down.
Fig. 4c shows a perspective side view of the implantable apparatus according to Fig. 4a, wherein the implantable apparatus is shown upside down.

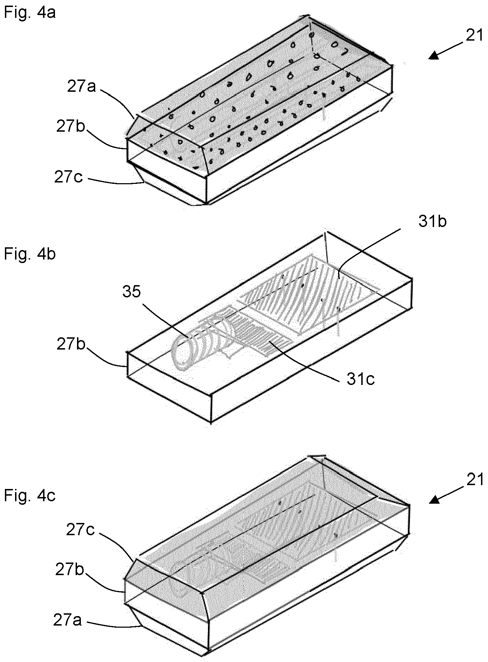

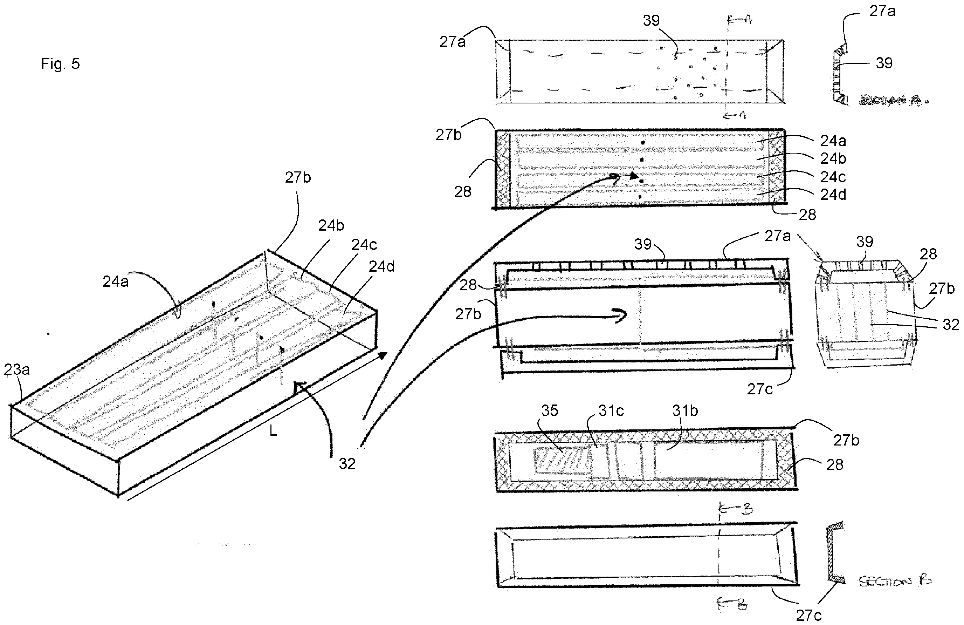

Fig. 5 shows, on the left side, a perspective view of the medial wafer substrate of the implantable apparatus according to Fig. 4a, and shows on the right of the page a collection of five vertically stapled drawings, from top to bottom: a top view of the first (top) wafer substrate of the implantable apparatus according to Fig. 4a, and also a cross sectional view taken along line A-A; a top view of the second (medial) wafer substrate of the implantable apparatus according to Fig. 4a, a cross sectional view taken along the length L and vertically through the center of the top surfaces of the wafer substrates of the implantable apparatus according to Fig. 4a, and also a cross sectional view taken along a line running along the through hole 32; a bottom view of the second (medial) wafer substrate of the implantable apparatus according to Fig. 4a, a bottom view on the bottom side of the third (bottom) wafer substrate of the implantable apparatus according to Fig. 4a, and also a cross sectional view taken along line B-B.
Fig. 6 shows a system according to the invention using an implantable apparatus according to the invention and a biosensor device according to the invention, respectively according to an example.
Fig. 7 shows a schematic of an embodiment of the electronic circuit of a biosensor device according to an embodiment of the invention.

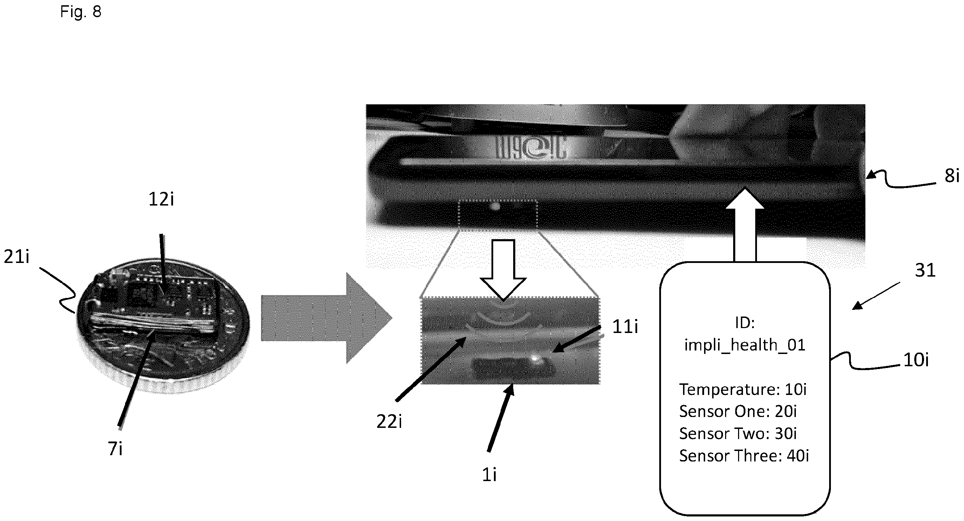

Fig. 8 illustrates the working principle of the biosensor device according to an embodiment of the invention.
Fig. 9 shows schematically the layout of a rigid PCB backside of the biosensor device according to an embodiment of the invention.
Fig. 10 shows schematically the layout of a rigid PCB frontside of the biosensor device according to an embodiment of the invention.
Fig. 11 shows three diagrams with measurement data measured by a biosensor device according to an embodiment of the invention.

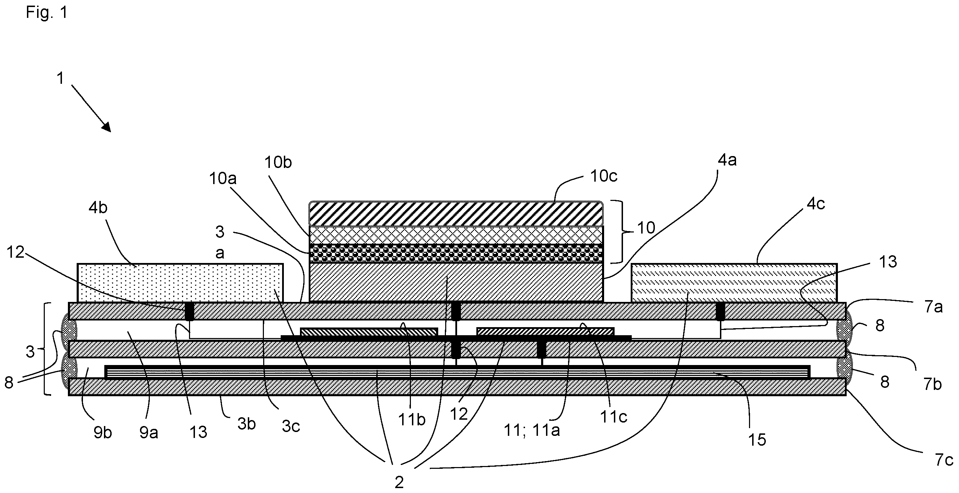

Fig. 1 shows a schematic cross sectional view of an implantable apparatus 1 according to an embodiment of the invention. The implantable apparatus 1 comprises a biosensor device 2 and an encapsulation device 3. The biosensor device 2 comprises all functional components needed for providing a working implantable apparatus, except the encapsulation device 3, which is specific for providing long-term operation of the biosensor device within living tissue and which provides biocompatibility for preventing or reducing the risk of rejection or adverse reactions due to various factors, including the body's immune response or due to release of harmful substances caused by degradation of the implantable apparatus. In the present case, the outer layer of the encapsulation device is substantially completely made from glass, except from the electrodes and conducting traces (not shown) running on the surface of the top wafer substrate 7a. The top wafer substrate 7a is part of the biosensor device 2 and additionally forms a part of the encapsulation device 3. In particular, the encapsulation device is made substantially completely from glass, and the electrodes are provided on the top surface 3a of the encapsulation device 3. The encapsulation device 3 is substantially in its entirety made from glass, in particular Borofloat^{®} by Schott AG, Germany. The top glass wafer 7a and the medial glass wafer substrate 7b, optionally also the bottom glass wafer substrate 7c, comprise vias 12 filled with a metal conducting material, e.g. Tungsten or NiFe. Such glass wafer substrates may be cut from larger wafers which ae commercially available as HermeS^{®} wafers from Schott AG, Germany. The three glass wafer substrates 7a, 7b, 7c are connected here by glass welding seams 8, which hermetically seal the cavities 9a and 9b inside the encapsulation device.

The electrodes 4a, 4b, 4c are sensing elements of the biosensor device 2 and are configured for performing voltammetry measurements, in particular DPV, when the implantable apparatus is implanted in the tissue. The electrodes include a counter electrode 4b attached on the top surface 3a of the glass wafer substrate 7a, which is a planar gold electrode, a reference electrode 4c attached on the top surface 3a of the glass wafer substrate 7a, which is also a planar gold electrode, and the working electrode 4a, which is also a planar gold electrode having a functionalized surface and being attached on the top surface 3a of the glass wafer substrate 7a. The functionalized surface is shown simplified as a stack 10 of layers. However, the real three-dimensional atomic surface structure (not shown) is more complex. Alternatively, another number of electrodes may be provided for allowing an electrical measurement, preferably, four electrodes may be provided.

Moreover, two or more electrodes may be provided, in general, on different surfaces of the implantable apparatus or the encapsulation device or the biosensor device, in particular, on any of the wafer substrates. For example, one or more electrodes may be provided on a first (outer) surface 3a of the encapsulation device, and one or more electrodes may be provided on a second (outer) surface 3b of the encapsulation device, which other second surface 3b may be opposing to the first surface 3a.

The stack 10 of layers results from the layer-wise or respectively, stepwise deposition of different nanoparticles or molecules on the previously existing surface. Thee stack 10 of layers is also referred to as the biosensor membrane 10.

The biosensor membrane 10 includes the layer of gold nanoparticles 10a, which is deposited on the planar gold surface of the electrode 4a. While in general, the gold nanoparticle layer 10a may be omitted, this layer is beneficial for improving the detection signal. The gold nanoparticles have an diameter of about 60 nm, while other particles sizes in the range between 10 nm and 200 nm, or between 30 and 100 nm, are also preferred. The effect of using gold layers is that the overall gold surface interacting with a body fluid, is generally enlarged compared with the planar surface of electrode 4a. In consequence, the surface provided by the nanoparticles and being available for binding the aptamers to the electrode 4a is significantly enlarged. As a result, the number of binding sites for binding the biomarker (hormons or other targets in a body fluid) is significantly increased and the signal quality, in particular the signal to noise ration of the measurement is improved.

On top of the gold nanoparticle layer 10a, an additional layer 10b of nanoparticles is provided, in the present case. The additional layer of metal nanoparticles 10b, which are platinum nanoparticles in the present case, was found to improve the lifetime and reliability of the biosensor membrane 10.

On top of the nanoparticle layers 10a, 10b, the aptamers 10c are deposited and bound to the nanoparticles, thereby completing the biosensor membrane 10. The thiolated aptamers tend to form a self-assembled monolayer (SAM) on the metal surface of the nanoparticles, which helps to improve the binding stability and the permanent attachment of the aptamer to the gold/platinum platform.

The biosensor was prepared in three steps:
1) Activation of the deposited gold layer on glass, this stage is done by connecting the working electrode (gold) to a potentiostat and applying continuous cyclic voltammetry (CV) in acid solution; this optional step of activating the gold electrode usually involves a cleaning or conditioning process to remove any contaminants or oxide layers, ensuring better electrical contact and performance.
2) Deposition of gold nanoparticles on the gold layer on top of the glass, and optionally, providing the platinum nanoparticles;
3) provision of the aptamer layer.

The electronic control device 11 here includes a printed circuit board 11a, which carries an integrated circuit 11b for controlling the electrodes 4a, 4b, 4c and for, respectively, controlling the electrical measurement of the at least one biological quantity. The electronic control device 11, in particular traces (not shown here) on the printed circuit board 11a leading to the integrated circuit 11b, is connected by wires 13 to the vias 12 leading to the electrodes. Moreover, the electronic control device 11 here includes the communication device 11c, which is an RFID device, in particular a NFC chipset, in the present case. The NFC chipset is connected to the integrated circuit 11b by a trace (not shown here) on the PCB 11a. Moreover, the NFC chipset is connected to the antenna 15, in the present case by a via 12 and a wire on the PCB 11a. The antenna 15 may be mounted anywhere at the biosensor 2 or respectively at the implantable apparatus 1, preferably inside the encapsulation device 3. Preferably, the antenna 15 is mounted at a wafer substrate or on a part of the encapsulation device 3, preferably at the same wafer substrate, which also carries the electronic control device 11.

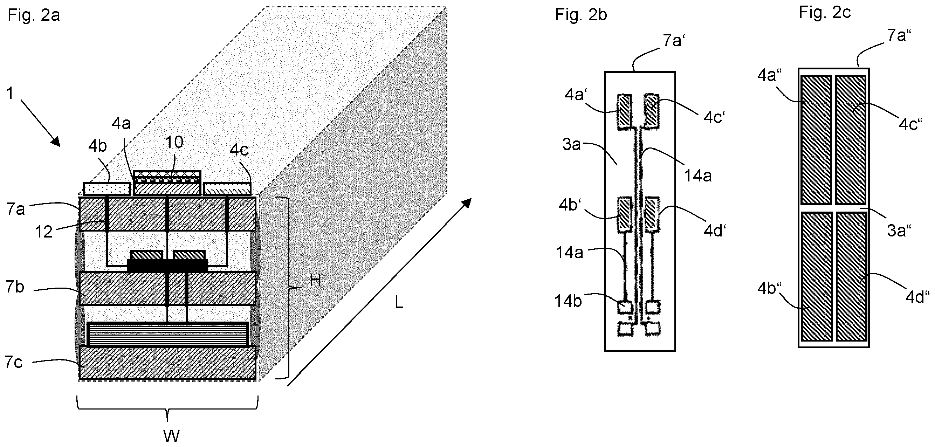

The electrodes 4a, 4b, 4c, are connected to the electronic control device by using the vias 12. The benefit of using a substrate wafer having the vias 12 is a much more compact setup of the biosensor device 2 and therefore, of the implantable 1. Each of the electrodes 4a, 4b, 4c may be connected to at least one via 12 by traces of gold, which may be provided on the gold surface and leading from the gold electrode to a gold connecting pad, see Fig. 2b. In Fig. 1, the electrode 4a is directly mounted to a via located beneath the electrode 4a, the same connection route is provided for the electrodes 4b and 4c.

The electronic control device 11 is attached here to a wafer substrate, here the medial wafer substrate 7b. However, it is, in general, also highly preferred that the electronic control device 11 may be attached to the same wafer substrate, which also carries at least one electrode, preferably on the side 3c of the wafer substrate being opposite to the side 3a, which carries the electrode.

The integrated circuit 11b is an ASIC, in the present case.

The electronic control device 11, in particular the integrated circuit 11b, is configured, in particular programmed, to provide an electrical measurement for obtaining a measurement signal or to repeatedly obtain a measurement signal for measuring the concentration of a biomarker in the body fluid, or the time dependent change of a signal in the body fluid, wherein the signal represents the amount of biomarkers binding to the biosensor membrane 10. The measurement may be triggered by a time schedule, or may be triggered by an external computer device, e.g. a smart phone having a control app installed, which in turn may be operated by a user (e.g. touching a "start"-area in a graphical user interface provided by the control app on the screen of a mobile phone device) to initiate a measurement.

The electrical measurement, preferably, is a voltammetry measurement, in particular a DPV measurement:

In general, DPV stands for Differential Pulse Voltammetry, which is an electrochemical technique used for signal detection and quantification in various applications. It involves applying a series of voltage pulses to the electrodes being in touch with the electrolyte forming the body fluid (e.g.: interstitial fluid) and measuring the resulting current response.

Electrode setup: A working electrode 4a, a reference electrode 4c, and a counter electrode 4b are provided. Alternatively, another number of electrodes may be provided for allowing an electrical measurement, preferably, four electrodes may be provided.

The DPV technique applies a series of voltage pulses to the working electrode 4a. The pulses typically consist of a step potential, a holding potential, and a pulse width. The step potential provides the driving force for an electrochemical reaction, and the holding potential allows time for the system to reach equilibrium before the next pulse.

Current measurement: After each voltage pulse, the resulting current response is measured using a potentiostat. The current response corresponds to the electrochemical reaction occurring at the working electrode.

The obtained current responses are typically plotted against the applied voltage pulses. The resulting DPV curve exhibits characteristic peak shapes that can provide information about the analyte concentration, redox reactions, and other electrochemical processes occurring in the system with the analyte of interest, facilitating the electrochemical reaction.

Fig. 2a shows a schematic perspective view of the implantable apparatus 1 according to Fig. 1, wherein this view gives an impression of the real dimensions of the implantable apparatus 1, which has a width W = 2.0 mm, a height H = 2.0 mm and a length L = 16 mm. The implantable apparatus has rounded edges and corners (not shown) and can be implanted by a surgeon using a syringe.

Fig. 2b shows a top view of the top surface of an electrode carrying wafer substrate 7a' of an implantable apparatus 1 according to an embodiment of the invention, which may be used in Fig. 1a, the wafer substrate 7a' having vias 12. The electrodes 4a', 4b', 4c', 4d' are deposited on the top surface 3a of the wafer substrate. Here, four electrodes are provided, wherein each electrode is connected to a connection pad 14b by a conductive trace 14a, both being deposited in the same fabrication step with the gold electrodes on the same surface 3a and being made from the same gold.

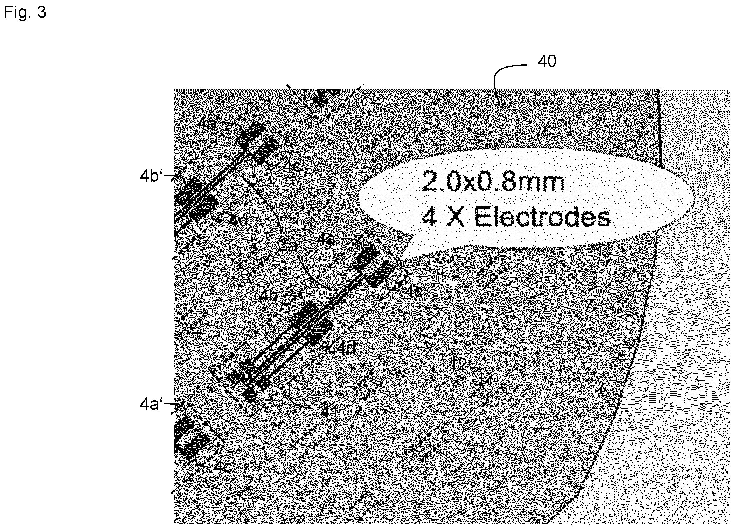

Fig. 3a shows a top view of a section of a round wafer 40 containing multiple electrode assemblies for cutting the same out from the wafer, for fabricating multiple electrode carrying wafer substrates 7a' as shown in Fig. 2b, each for an implantable apparatus according to an embodiment of the invention. The wafer substrate 40 is provided with assemblies of vias 12, which run perpendicular through the wafer surface and which are filled with conductive metal, e.g. Tungsten. The wafer 40 is a HermeS^{®} glass wafer from Schott AG, Germany.

Fig. 2c shows a top view of the top surface of another electrode carrying wafer substrate 7a"of an implantable apparatus according to another embodiment of the invention. The wafer 40 is a HermeS^{®} glass wafer from Schott AG, Germany. The four electrodes 4a", 4b", 4c", 4d" are deposited on the top surface 3a" of the wafer substrate 7a". The four wafer substrates cover almost the entire surface of the wafer substrate, or respectively, cover a fraction of the surface, the fraction being chosen from the range 80 to 100%, preferably 90 to 100%, preferably 90 to 99%. Using a larger surface for the electrodes results ini an improved signal quality when running the electrical measurements, because a larger number of binding sited for binding the biomarker can be provided.

Fig. 4a shows a perspective side view of an implantable apparatus 21 according to an embodiment of the invention being made of a top wafer substrate 27a, a medial wafer substrate 27b and a bottom wafer substrate 27a. The top wafer substrate 27a and the bottom wafer substrate 27a are configured having a trough shape and being arranged as covers for covering the parts respectively located on the top side and the bottom side of the medial wafer substrate 27b.

Fig. 4b shows a perspective side view of the medial wafer substrate 27b of the implantable apparatus according to Fig. 4a, wherein the medial wafer substrate is shown upside down. On the bottom side of the medial wafer substrate 27b, multiple parts of the electronic control device of the biosensor device of the implantable apparatus 21 are attached: the integrated circuit (ASIC) 31b for electrically controlling the electrodes 24a, 24b, 24c, 24d, the communication apparatus 31c being an NFC device and including the NFC antenna 35.

In analogy to the view of the medial wafer substrate 27b in Fig. 4b, Fig. 5 shows, on the left side, a perspective top view of the medial wafer substrate 27b of the implantable apparatus according to Fig. 4a, having four gold electrodes 24a, 24b, 24c, 24d being aligned in parallel and in a distance to each other along the length L of the medial wafer substrate 27b. The electrodes occupy almost the entire surface of the medial wafer substrate 27b. The medial wafer substrate 27b is a HermeS^{®} glass wafer from Schott AG, Germany, which has vias 32 running perpendicularly to the top surface through the wafer substrate and being filled with conductive metal, e.g. Tungsten. Each electrode is contacted to a via 32 and is connected by the via 32 to the ASIC 31b, which may be deposited directly on the bottom surface of the medial wafer substrate 27b, without using PCB for placing the electronic components, or alternatively, by using a PCB (not shown) for placing the electronic components. The use of the wafer having vias allows to implement the compact solution for an implantable apparatus according to this embodiment according to the invention. Features of the implantable 21 may be easily modified by the person skilled in the art with other preferred features and configurations as disclosed herein.

Fig. 5 shows, on the right of the page, a collection of five vertically stapled drawings, from top to bottom:
i. a top view of the first (top) wafer substrate 27a of the implantable apparatus 21 according to Fig. 4a, and also a cross sectional view taken along line A-A, which shows through-holes or pores 39. The first (top) wafer substrate 27a acts as a cover lid for covering the surface 23a carrying the four gold electrodes 24a, 24b, 24c, 24d. One or two of said electrodes carry a biosensor membrane, for example the biosensor membrane 10 as described with reference to Fig. 1. The other electrodes are gold electrodes used as reference or counter electrodes for the voltammetry (in particular: DVP) measurement. Using the cover lid 27a, the biosensor membrane and the electrodes are protected against mechanical damages, which may result from the implantation process or from the long-term use in a patient's body. The porous membrane formed by the main wall of the top wafer substrate and cover lid 27a allows an easy uptake of the body fluid inside the inner volume included by the trough shaped cover lid 27a and the top surface of the medial wafer substrate 27b, to which the cover lid 27a is welded, thereby firmly and non-releasable connecting the cover lid to the medial wafer substrate. The main wall of the cover lid, and possibly also its flanges surrounding the outer rim of the main wall, are made, as an example, from CoralPor^{®} porous glass, commercially available from Schott AG, Germany. The welding is achieved by laser welding, in particular femtolaser welding, at the welding lines and welding areas denoted by numeral 28, when the wafer substrates are all readily equipped with, for example, the electronic parts and the electrodes plus the biosensor membrane, and precisely aligned to each other.
ii. a top view of the second (medial) wafer substrate 27b of the implantable apparatus according to Fig. 4a; the vias 32 are shown here as black points for illustrative reasons, while they are in practice not visible because being covered by the Gold electrodes.
iii. a cross sectional view taken along the length L and vertically through the center of the top surfaces of the wafer substrates of the implantable apparatus according to Fig. 4a, showing in particular the via 32 running through the complete height of the medial wafer substrate for electrically connecting the top side and the bottom side; and also shown on the right, a cross sectional view taken along a line running along the through hole 32;
iv. a bottom view of the second (medial) wafer substrate of the implantable apparatus according to Fig. 4a, the electronic components 35, 31b and 31c being attached thereto; and
v. a bottom view on the bottom side of the third (bottom) wafer substrate 27c of the implantable apparatus according to Fig. 4a, and also a cross sectional view taken along line B-B. The wafer substrate 27c is a glass substrate formed with a through shape for being formed to cover the (electronic) components attached to the bottom surface of the medial wafer substrate 27b, to which the cover lid 27c is welded at the welding sites 28, using a femto-laser welding process. The welding creates a hermetical closure of the cavity between the cover lid 27 and the bottom surface of the medial wafer substrate 27b. Due to the hermiticity, no body fluid will enter the cavity and the electronic components will be reliably protected against corrosion, and the patients body is reliably protected against any non-biocompatible material of the biosensor during the whole lifetime of the implantable apparatus, which is reliably encapsulated by thee encapsulation device formed by the three wafer substrates 27a, 27b and 27c. With regard to this embodiment, no body part of a patient will be directly contacted by another material than the biocompatible glass, which forms the encapsulation device.

Description of a preferred example of the method of fabricating an implantable apparatus according to the invention:
Each biocompatible glass layer is prepared and cleaned in clean-room conditions.

The encapsulation device, preferably, uses one or more, preferably two or more, in particular three or four or up to four wafer substrates to layer the electrodes plus the biosensor membrane, in particular the electronic control device and preferably an NFC-antenna, and each wafer is aligned to a datum reference point and calibrated to aligned stacking.

A femtosecond laser is used to pass over in controlled phase planes to allow for internal stitching and welding of glass layers to create at least one cavity sealed to hermeticity. This sealed cavity, preferably, is capable to sustain 5 atmospheres (~550kPa) for 4 hours, with 2 hours dwell time and preferably not have a mass-spectrometer measured leak rate of more than 5 x10-8 atm.cm 3/s).

A femtosecond laser creates microzones of stress, each wafer design and chip geometry requires a unique boundary layer to allow for multiple cavities to be sealed on one chip. This improves overall yield of the biocompatible glass wafers and improves manufacturing efficiency. This process and design guideline is suitable for the glass wafer manufacturing process to allow for the singulated chips cut from the wafer with sealed cavities to be free from stress induced by the working heat-affected zone within the stitched areas.

Preferably, multiple wafers (two or more) containing a plurality of wafer substrates, for fabricating multiple implantable apparatuses in parallel, are aligned in a stack, then welded at the stitching points or areas, and then the resulting stack of welded glass wafer substrates, including at least one of the components (electrodes, electronic control device, in particular microcontroller, amplifier(s), RFID (preferably: NFC) chipset; antenna) enclosed in one or more cavities, are cut from the stacked and welded wafers.

Once the layers are welded, a quality assessment is completed on each chip to ensure both hermeticity and alignment of the internal working components of the device are within performance requirements.

This laser seal allows for a biocompatible sealed cavity that can be implanted for the full internal operation of the electronics to amplify the signals generated by the biosensor.

In a preferred embodiment of a method for preparing a glass encapsulation device, the method comprises one or more, or each, of the following steps:
- Each biocompatible glass layer is prepared and cleaned in clean-room conditions.
- The device uses up to four wafers to layer the electronics, biosensor and NFC-antenna and bio-membrane, and each wafer is aligned to a datum reference point and calibrated to aligned stacking.
- A femtosecond laser is used to pass over in controlled phase planes to allow for internal stitching and welding of glass layers to create a cavity sealed to hermeticity.
- This sealed cavity must sustain 5 atmospheres (~550kPa) for 4 hours, with 2 hours dwell time and not have a mass-spectrometer measured leak rate of more than 5 x10-8 atm.cm 3/s).
- A femtosecond laser creates microzones of stress, each wafer design and chip geometry requires a unique boundary layer to allow for multiple cavities to be sealed on one chip. This improves overall yield of the biocompatible glass wafers and improves manufacturing efficiency. This glass wafer manufacturing process allows for the singulated chips (stacked wafer substrates) cut from the wafer with sealed cavities to be free from stress induced by the working heat-affected zone within the stitched areas.
- A chip singulation process is done after the individual cavities are laser welded and is completed by a standard glass cleaving action, where the glass is cleaved along a scribing line and the heat affected zone.
- Once the layers are welded, a quality assessment is completed on each chip to ensure both hermeticity and alignment of the internal working components of the device are within performance requirements.
- This laser seal allows for a biocompatible sealed cavity housing the electronics that can be implanted for the full internal operation of the electronics to amplify the signals generated by the biosensor.
- The process of laser welding preferably completely seals the electronics within a glass cavity. This cavity has the exemplarily dimensions of 16x1.65x0.55mm. The electronics are routed to the aforementioned biosensor through vias, also known as TGVs with trace routings on the glass surface. The TGVs are hermetically sealed and allow for electrical contact with the biosensor electrodes on the external surface.

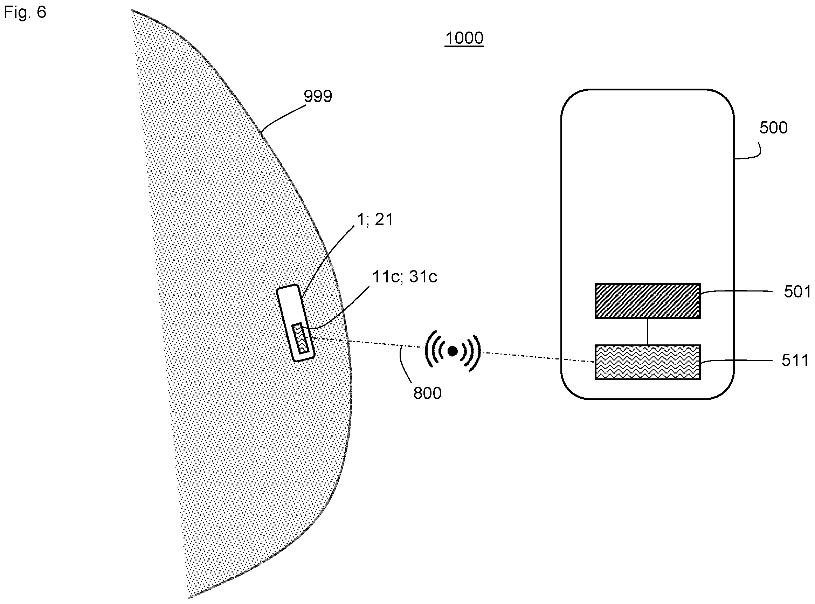

The details Fig. 6 shows a system 1000 for sensing at least one hormone in a body fluid inside the body 999 of a patient, comprising
- the subcutaneous implantable apparatus 1 or 21, which additionally comprises a communication device 11c or 31c, in particular NFC (Near Field Communication) device for wireless data communication with an external computer device 500 being separate from the implantable apparatus, and
- the external computer device 500, in particular a portable computer device, preferably a smart phone or tablet PC, comprising a CPU 501 for executing a computer program code, in particular the computer program code according to the invention, and a communication device 511, in particular NFC device, for wireless data communication with the implantable apparatus.

The data exchange is initiated, preferably, when the program code, for example an application executed by the CPU on the computer device, receives a user input via the touch screen (not shown) of the computer device 500, which sends a request to the NFC device 11c, 31c to transfer the measurement data, preferably in an encrypted form, to the computer device 500. Preferably, a measurement performed by the biosensor device 2 of the implantable 1 is initiated, preferably, when the program code, for example an application executed by the CPU on the computer device, receives a user input via the touch screen (not shown) of the computer device 500, which sends a request to the NFC device 11c, 31c to initiate the measurement. Since the implantable preferably is a battery-less device, the radio waves of the communication device 501 are preferably used to provide the energy required for the measurement/and or for the encryption of the measurement data and/or for transferring the measurement data to the computer device via the wireless connection 800.

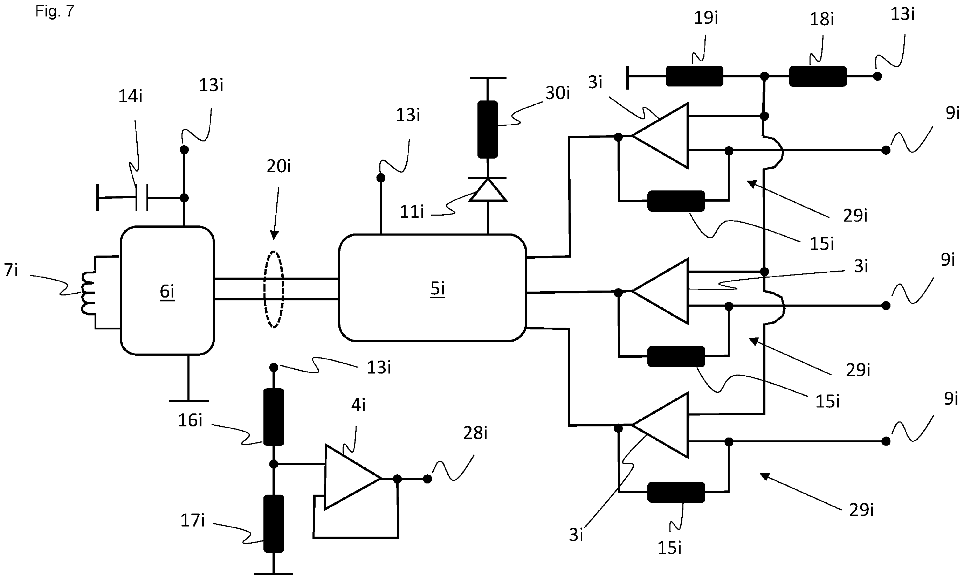

Fig. 7 shows a schematic of an embodiment of the electronic circuit. The electronic circuitry embedded inside the implantable apparatus is composed by 4i off-the-shelf components as shown in Fig. 7. These four components comprise the central microcontroller 5i (MCU), which is an ultra-low powered microcontroller with 8-bit architecture, such as the ATTiny20 and PIC12LF device families from Microchip, Chandler, AZ, USA. Further, the NFC chip tag 6i, for example the NTAG I2C plus family from NXP, Eindhoven, The Netherlands and finally, two operational amplifiers 3 with dual amplification channels, having very low input bias current, for example 1 pA and ultra-low current consumption, for example 20 µA, such as the AD8506 family from Analog Devices, Wilmington, MA, USA. The maximum current consumption of the electronic circuit 2i is set at a level of 1 mA, with minimal voltage level of 1.8 V, thereby yielding a power consumption of 1.8 mW. Three electrochemical analytes or species can be monitored simultaneously by the available working electrode pads 9i that connect to three independent amplifiers 3i mounted in a transimpedance circuit topology 29i to convert and amplify the ionic currents into a voltage equivalent level by means of resistors 15i in the schematics as shown in Fig. 7, and relative to the reference voltage level imposed by the reference electrode 28i. Then, the output voltage signal of each amplifier 3i is digitized in three separate analogue-to-digital converters (ADC) internal to the microcontroller 5i at a rate of 24 samples per second and resolution of 10-bit. An additional amplifier 4i is responsible to generate the aforementioned voltage reference level during the amperiometric measurements by means of a voltage divider circuit topology centered around resistors 16i and 17i, followed by signal buffering by the amplifier 4i. A similar voltage divider topology is employed at the positive terminal of each transimpedance amplifier 29i to generate an internal bias voltage that level shifts the amplification baseline above the ground signal, achieved by resistors 18i and 19i.

The data samples originated during the digitization stage are temporarily stored inside the RAM memory of the MCU 5i, digitally processed and encrypted by a lightweight security algorithm before being digitally transmitted to the NFC chip tag 6i using the standard I2C interface 20i protocol at a speed of 9600 bps (baudrate), where they are internally stored inside the non-volatile memory.

Although the I2C communication protocol was originally developed by Philips Semiconductors, which cover the definition of the communication signals (SDA and SCL), and the specifications for the transmission baudrate and byte format, the data packet (byte stream) exchanged between the MCU and NFC chip tag is completely custom-made in here, with some of the transmission bytes encoding sequence actions that the MCU 5i needs to perform, for example acquiring the biosignals, i.e. the current, and data transmission or control parameters for the operation of the implant 1i itself. Whilst the harvested voltage level 13i can be generated by any NFC-enabled device 8i, or any RF source with the same frequency, the MCU 5i will not start any operation until receiving a valid command sequence from an authorized mobile phone running the respective app.

Fig. 2 illustrates the working principle of the biosensor device 1i. The biosensor device 1i is an implantable apparatus and comprises an encapsulation device according to the invention being substantially made from glass. The biosensor device 1i is shown positioned on top of a coin 21i for representation of the proportions. The NFC antenna 7i is shown wrapped around the perimeter of the PCB board. A small, embedded light emitting diode (LED) 11i gives visual feedback of the operations occurring at the implantable side when successfully powered up by the NFC interface of the mobile phone 8i. In the presence of an external NFC field 22, the internally stored and encrypted data samples are wirelessly transmitted to the external mobile phone 8i (uplink) and visualized on a dedicated application software 31i of installed on the phone 8i with information about temperature levels also being provided through the uplink communication channel as the microcontroller 5i also possesses an internal temperature indicator built-in on the chip die. By the same process, the AC radiofrequency waves 22i generated by the mobile phone 8 are continuously captured by the internal NFC antenna 7i. The antenna 7i is made of 20 turns of enamelled copper wire with thickness of 0.15 mm and wrapped around the external limits of the circuit board 12i, having the dimensions of, for example 6 mm x 12 mm, and converted to an equivalent DC level 13 (V_{HARVEST}) by the NFC chip tag 6i itself, before making it available to the rest of the embedded electronics. On the opposite direction of communication (or downlink), specific data 10i, for example user parameters can be sent from the same mobile phone's application software 31 ii to set up ID numbers to the implantable biosensor device 1i, for example device operation and control of the amperometric measurement process.

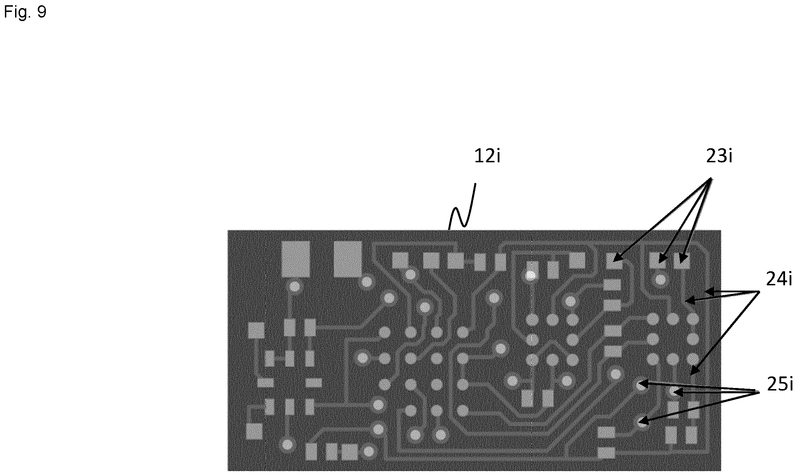

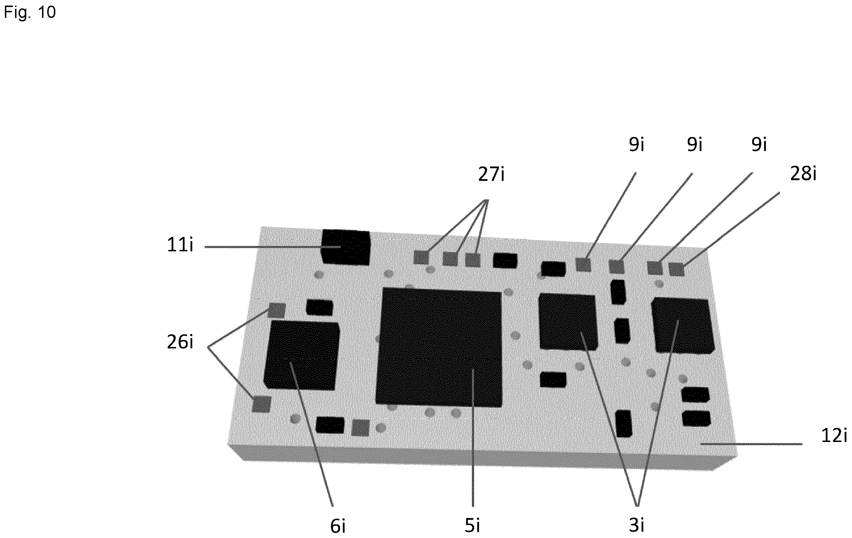

In Fig. 9 the layout of a rigid PCB backside of the biosensor device is shown schematically, and in Fig. 10 a 3D schematic layout of a rigid PCB frontside of the biosensor device is shown. Physically, the described electronic schematic of the implantable apparatus is translated into printed-circuit-board (PCB) technology, with the protected trace conductors 24i and through-layer vias 25i, which are Vertical Interconnected Accesses, i.e. a conductive pathway or hole that connects different layers of, e.g. a PCB or IC together, thereby providing means for electric current to pass through the layers of the circuit board, carrying the electrical signals between the different chipsets, resistors 15i - 19i, 30i, capacitors 14i and NFC antenna 7i, whereas exposed electronic pads 23i made of an Ni/Au surface finishing process are used to solder the components to the PCB 12i substrate or left uncovered 27i to allow connection to programming pins for the MCU 5i or electrochemical functionalization of the electrochemical sensors, as shown in both Figs. 9 and 10. The copper traces 24i are placed both on the TOP and BOTTOM layers of the PCB 12i, whereas component assembly only occurs on the TOP layer. Rigid 12i, for example laminated copper in FR-4 circuit board plus epoxy resin, or flexible 12i, for example polyimide film substrates can be used for the fabrication of the described PCB 8i.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method.

Reference throughout this specification to "an embodiment" or "the embodiment" or "preferred configuration" means that a particular feature, structure, or characteristic described in connection with that embodiment is included in at least one embodiment. Thus, the quoted phrases, or variations thereof, as recited throughout this specification are not necessarily all referring to the same embodiment.

Recitation in the claims of the term "first" with respect to a feature or element does not necessarily imply the existence of a second or additional such feature or element. Changes may be made to the details of the above-described embodiments without departing from the underlying principles of the present disclosure.

Further, it should be appreciated by one of skill in the art with the benefit of this disclosure that in the above description of embodiments, various features are sometimes grouped together in a single embodiment, figure, or description thereof for the purpose of streamlining the disclosure. This method of disclosure, however, is not to be interpreted as reflecting an intention that any claim requires more features than those expressly recited in that claim. Rather, as the following claims reflect, inventive aspects lie in a combination of fewer than all features of any single foregoing disclosed embodiment. Thus, the claims are hereby expressly incorporated into the present description, with each claim standing on its own as a separate embodiment. This disclosure includes all permutations of the independent claims with their dependent claims.

## Claims

1. Biosensor device for sensing at least one hormone, comprising
(a) at least one metal electrode surface; and
(b) a biosensor membrane including at least one aptamer, which is attached to the metal electrode surface, wherein the aptamer:
(i) is capable of binding the at least one hormone; and
(ii) is modified with one or more functional group for attaching the at least one aptamer to the metal electrode surface.

2. The biosensor device of claim 1, configured for sensing the at least one hormone in a body fluid, in particular an interstitial fluid.

3. The biosensor device of any of the preceding claims, wherein the at least one hormone is selected from the group consisting of estradiol, luteinizing hormone (LH), progesterone, and any combination thereof.

4. The biosensor device of any of the preceding claims, wherein the biosensor device further comprises a carrier, particularly a glass carrier, for example a wafer substrate, for carrying the at least one metal electrode surface.

5. The biosensor device of any of the preceding claims, wherein the aptamer is a single stranded nucleic acid molecule, preferably a DNA or RNA molecule.

6. The biosensor device of any of the preceding claims, wherein the aptamer has a length of about 25 to 70 nucleotides, preferably about 30 to about 65 nucleotides.

7. The biosensor device of any of the preceding claims, wherein the functional group for attaching the at least one aptamer to the metal electrode surface is a thiol group, which is, preferably, present at the end of the aptamer.

8. The biosensor device of any of the preceding claims, wherein the aptamer is a single stranded nucleic acid molecule, preferably a DNA or RNA molecule, and the functional group for attaching the at least one aptamer to the metal electrode surface is present at the 3' end or 5' end, preferably the 5' end.

9. The biosensor device of any of the preceding claims, wherein the aptamer is capable of binding at least one hormone selected from the group consisting of estradiol, luteinizing hormone (LH), and progesterone.

10. The biosensor device of any of the preceding claims, wherein the aptamer is one or more selected from group consisting of SEQ ID NO: 1, SEQ ID NO: 2, SEQ ID NO: 3, and SEQ ID NO: 4.

11. The biosensor device of any one of claims 1 to 10 for use in diagnosis, particularly in sensing at least one hormone, more particularly for monitoring hormones during assisted reproductive technology (ART), during menopause, in a hormone disorder such as polycystic ovary syndrome (PCOS), in endometriosis and/or during hormone treatment.

12. Implantable apparatus for being implanted, in particular subcutaneously implanted, into the body of an animal or human, comprising
• a biosensor device according to any of the previous claims, and
• an electronic control device for controlling the biosensor device, in particular for generating, collecting, and preferably, encrypting, measurement data obtained by the sensing of the biosensor device.

13. System for sensing at least one hormone, comprising
the implantable apparatus of claim 12, which additionally comprises a communication device, in particular NFC (Near Field Communication) device for wireless data communication with an external computer device being separate from the implantable apparatus, and
the external computer device comprising a communication device, in particular NFC device, for wireless data communication with the implantable apparatus.

14. Use of the biosensor device of any one of claims 1 to 11 for *in vitro* diagnosis, particularly in sensing of at least one hormone.

15. Method for operating a biosensor device according to any of the claims 1 to 11 or the implantable apparatus of claim 12 or the system of claim 13, comprising the steps:
(a) contacting the biosensor membrane with a body fluid, particularly interstitial fluid, which contains the at least one hormone;
(b) allowing the at least one hormone to bind to the at least one aptamer of the biosensor membrane; and
(c) detecting the binding of the at least one hormone by electrically controlling the at least one metal electrode surface.
